# FASCICULE DE BREVET EUROPEEN

(11) **EP 1 904 847 B1**
(45) Date de publication et mention de la délivrance du brevet: **16.09.2009**
(21) Numéro de dépôt: 06778902.4
(22) Date de dépôt: 21.07.2006
(51) Int. Cl.: G01N 33/542

(54) **SUBSTRATS FLUORESCENTS SACCHARIDIQUES, LEUR PROCEDE DE PREPARATION ET LEURS UTILISATIONS**
SACCHARID-FLUORESZENZSUBSTRATE, HERSTELLUNGSVERFAHREN UND VERWENDUNGEN DAVON
SACCHARIDE FLUORESCENT SUBSTRATES, PREPARATION METHOD AND USES THEREOF

(30) Priorité: 21.07.2005 FR 0507759
(43) Date de publication de la demande: 02.04.2008
(73) Titulaire: COMMISSARIAT A L'ENERGIE ATOMIQUE, 75015 Paris (FR); INSTITUT NATIONAL DE LA SANTE ET DE LA RECHERCHE MEDICALE (INSERM), 75013 Paris (FR); Centre National de la Recherche Scientifique, 75016 Paris (FR)
(72) Inventeur: TEXIER-NOGUES, Isabelle, F-38000 Grenoble (FR); ROBERT, Véronique, F-38000 GRENOBLE (FR); COLL, Jean-Luc, F-38640 Claix (FR); IMBERTY, Anne, F-38640 Claix (FR)
(74) Mandataire: Goulard, Sophie
(86) Numéro de dépôt international: PCT/FR2006/001786
(87) Numéro de publication internationale: WO 2007/010145

(56) Documents cités:
- EP-A- 0 731 178
- US-A1- 2004 053 287
- COTTAZ S ET AL: "A fluorescence-quenched chitopentaose for the study of endo-chitinases and chitobiosidases." EUROPEAN JOURNAL OF BIOCHEMISTRY / FEBS. SEP 2000, vol. 267, no. 17, septembre 2000 (2000-09), pages 5593-5600, XP002377041 ISSN: 0014-2956 cité dans la demande
- MATSUOKA K ET AL: "A bi-fluorescence-labeled substrate for ceramide glycanase based on fluorescence energy transfer" CARBOHYDRATE RESEARCH, ELSEVIER SCIENTIFIC PUBLISHING COMPANY. AMSTERDAM, NL, vol. 276, no. 1, 16 octobre 1995 (1995-10-16), pages 31-42, XP004021862 ISSN: 0008-6215
- GIORGIO ET AL: "Theoretical simulation of the electronic circular dichroism spectrum of calicheamicin" BIOORGANIC & MEDICINAL CHEMISTRY, ELSEVIER SCIENCE LTD, GB, vol. 13, no. 17, 29 avril 2005 (2005-04-29), pages 5072-5079, XP005045017 ISSN: 0968-0896
- TAKEUCHI M ET AL: "Sugar Building-Block as a Fine-Tunable Link for Electron-Donor - Electron-Acceptor Couples" TETRAHEDRON LETTERS, ELSEVIER, AMSTERDAM, NL, vol. 40, no. 19, 7 mai 1999 (1999-05-07), pages 3745-3748, XP004163821 ISSN: 0040-4039
- BERLIER ET AL: "Quantitative comparison of long-wavelength alexa fluor dyes to cy dyes: fluorescence of the dyes and their bioconjugates" THE JOURNAL OF HISTOCHEMISTRY & CYTOCHEMISTRY, vol. 51, no. 12, 2003, pages 1699-1712,
- [Online] extrait de SIGMA-ALDRICH Database accession no. 93662

## Description

La présente invention est relative à des substrats enzymatiques fluorescents de nature saccharidique comportant sur la même unité saccharidique un fluorophore F₁ et un inhibiteur de la fluorescence de F₁, à leur utilisation pour la préparation d'un réactif de diagnostic pour l'imagerie fonctionnelle *in vivo,* ainsi qu'au réactif de diagnostic pour l'imagerie fonctionnelle renfermant au moins un tel substrat enzymatique.

La fluorescence est une technique très utilisée pour la détection d'activités enzymatiques *in vitro.* C'est une technique peu coûteuse, rapide et généralement très sensible.

De nombreuses enzymes d'importance biologique significative ont pour substrats des dérivés saccharidiques. En effet, des gènes rapporteurs exprimant différentes enzymes telles que la β-galactosidase (β-gal), la β-glucuronidase (β-glu), le chloramphénicol, l'acétyltransférase, la luciférase, les protéines fluorescentes telles que la "Green Fluorescent Protein" (GFP), sont très utilisés aujourd'hui en biologie pour étudier l'expression de gènes (transcription et traduction de l'ADN en protéines), la transfection, ou d'autres processus biologiques. Les gènes rapporteurs peuvent servir de témoins pour démontrer l'introduction et la transcription d'un autre gène d'intérêt, situé sur la même partie codante de l'ADN. Les constructions d'ADN contenant les gènes rapporteurs sont introduites dans l'animal pour former des animaux transgéniques. Par exemple, le nombre de souris transgéniques déjà construites est très important et en augmentation rapide.

Dans un très grand nombre de cas, le gène marqueur utilisé est le gène *lacZ* qui code pour la β-gal de *E.coli.* Un autre exemple de gène marqueur également utilisé est le gène gusA qui code pour la β-glu de *E. coli.* Or les substrats des enzymes exprimées par certains de ces gènes, et en particulier par les gènes *lacZ* et *gusA,* sont des dérivés saccharidiques. Il est donc très important de pouvoir disposer de substrats saccharidiques pour pouvoir détecter l'activité de ces enzymes.

De nombreux substrats de nature saccharidique existent déjà pour détecter des activités enzymatiques telles que par exemple les activités enzymatiques de β-gal et β-glu. Ces substrats enzymatiques peuvent notamment être:
- des substrats pour l'imagerie nucléaire,
- des substrats chimiluminescents tels que les substrats commercialisés sous les dénominations commerciales Lumi-Gal ® 530 par la société Lumigen Inc. (USA) et Galacton-*Star*® par la société Applied-Biosystems (USA) ;
- des substrats pour la détection diélectrophorétique,
- des substrats pour l'IRM,
- des substrats formant des précipités,
- des substrats pour dosages spectrophotométriques dont le substrat X-gal (5-bromo-4-chloro-3-indolyl-beta-D-galactopyranoside) vendu par exemple sous la dénomination commerciale *Blue*Tech ® par la société Mirador DNA Design Inc. ; et
- des substrats fluorescents.
   Idéalement, ces substrats doivent avoir les propriétés suivantes :

- une cinétique de réaction enzymatique rapide,
- une constante de Michaelis faible,
- une grande différence entre les propriétés d'intérêt du substrat et celles de son(ses) produit(s) (propriétés d'intérêt : absorption pour un substrat chromogénique, fluorescence pour un substrat fluorogénique, etc...).

L'intérêt des substrats fluorescents, par rapport aux autres substrats décrits ci-dessus, est leur sensibilité de détection et le faible coût de l'instrumentation nécessaire pour les utiliser. Ils permettent, au même titre que l'IRM, et dans certaines conditions, de réaliser une détection enzymatique *in vivo.*

D'une manière générale, les substrats enzymatiques fluorescents fonctionnent sur le principe suivant : un substrat non fluorescent dans la gamme de longueur d'onde de détection donne un produit fluorescent dans cette même gamme de longueur d'onde lorsqu'il est mis en présence d'une enzyme dont on souhaite détecter l'activité et qui est spécifique du substrat utilisé. Il est donc nécessaire de trouver des fluorophores dont la fluorescence est inhibée initialement lorsqu'ils sont greffés sur le substrat et qui est capable de se libérer après réaction avec l'enzyme dont on souhaite détecter l'activité. Le choix des fluorophores disponibles commercialement se trouve donc limité par cette contrainte d'inhibition initiale de la fluorescence lorsque le fluorophore est fixé sur le substrat enzymatique.

*In vivo,* le développement récent des méthodes optiques ouvre de nouveaux horizons pour l'imagerie fonctionnelle. Il est maintenant possible de suivre en temps réel et de façon non invasive, l'expression de gènes chez des animaux, en particulier chez la souris, après anesthésie. L'imagerie optique présente un certain nombre d'avantages par rapport aux autres techniques d'imagerie fonctionnelle telles que l'imagerie par résonance magnétique (IRM), l'imagerie par tomographie d'émission de positons (TEP) et l'imagerie par émission monophotonique (SPECT) :
- elle évite la manipulation de molécules radioactives, écartant ainsi les contraintes et les risques qui y sont liés (radioprotection, gestion des déchets, source synchrotron pour les marqueurs TEP) ;
- elle ne nécessite pas de gros investissement d'instrumentation ;
- elle présente une bonne sensibilité par rapport à l'IRM, en terme de quantité de marqueur injectée.

L'imagerie optique met en oeuvre des substrats enzymatiques fluorescents.

Lorsque l'on souhaite détecter la présence d'une activité enzymatique *in vivo,* par exemple chez un petit animal de laboratoire comme la souris, très peu de molécules fluorescentes sont disponibles pour cette application. En effet, pour que la lumière d'excitation et la lumière émise par le fluorophore puissent traverser les tissus, il convient d'utiliser des fluorophores absorbant et émettant dans le proche infrarouge, c'est-à-dire à une longueur d'onde comprise entre 640 et 900 nm. Or, on trouve très peu de molécules fluorescentes dans ce domaine de longueurs d'onde actuellement disponibles commercialement (limitation aux cyanines essentiellement). La double contrainte, à savoir l'inhibition initiale de la fluorescence lorsque le fluorophore est fixé sur le substrat et l'utilisation d'un fluorophore absorbant et émettant dans le proche infrarouge, est sans doute à l'origine de l'absence de substrat enzymatique fluorescent de nature saccharidique dans ce domaine de longueurs d'onde.

En effet, la majorité des substrats enzymatiques fluorescents de nature saccharidique actuellement disponibles sur le marché n'est pas construite à partir de groupements fluorophores absorbant et émettant dans le proche infrarouge. Il est par exemple possible de se procurer :
- des substrats à base de fluorescéine pour la détection de l'activité β-gal parmi lesquels on peut par exemple mentionner le FDG (fluorescein-di-β-D-galactopyranoside) (excitation 490 nm / émission 514 nm) ou l'un de ses dérivés ;
- des substrats à base de coumarines ou d'umbelliferones pour la détection des activités β-gal, β-glu ou phosphatase, tels que les substrats MUG (4-méthylumbelliferone β-D-galactopyranoside), DiFMUG (6,8-difluoro-4-méthylumbelliferyl β-D-galactopyranoside), MUP (4-méthylumbelliferone phosphate), DiFMUP (6,8-difluoro-4-méthylumbelliferyl phosphate) et dérivés (excitation 350-380 nm / émission 450-470 nm) ; ou encore
- des substrats à base de résorufine et dérivés, notamment pour la détection de la lipase (excitation 570 nm / émission 585).

La plupart des substrats fluorescents commercialisés actuellement fonctionnent selon le principe représenté sur le Schéma A ci-après :

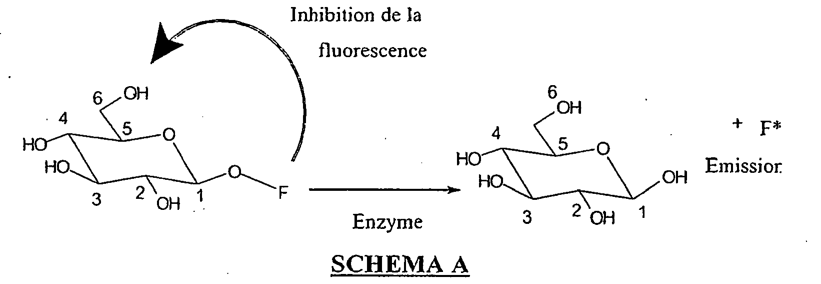

Sur ce schéma, le fluorophore F est greffé en position anomérique 1 (liaison anomérique de configuration β) sur un monosaccharide, le β-glucopyranose, pour former le substrat enzymatique. Ce substrat doit être faiblement fluorescent avant la réaction avec l'enzyme. Les groupements fluorophores doivent donc être choisis de telle sorte que leur fluorescence puisse être inhibée initialement par le monosaccharide. La réaction enzymatique induit un clivage de la liaison anomérique et libère le groupement fluorophore. Lorsque le groupement fluorophore est éloigné du monosaccharide, sa fluorescence n'est plus inhibée et il peut alors émettre un signal qui est détecté à l'aide d'un spectrofluorimètre. Le signal émis reflète l'activité enzymatique et est, dans un certain domaine de concentrations, proportionnel à la concentration en enzyme.

Les substrats fonctionnant selon le principe reporté sur le Schéma A présentent cependant un certain nombre d'inconvénients :
- le choix du groupement fluorophore est limité par le fait que sa fluorescence doit pouvoir être inhibée par son greffage sur le sucre ; tous les groupements fluorophores n'ont pas cette propriété, en particulier dans le domaine du proche infrarouge lorsque l'on souhaite pouvoir faire une détection *in vivo* (voir ci-avant) ;
- si l'inhibition de la fluorescence par le sucre n'est pas complète, la sensibilité de détection du système est médiocre. Pour remédier à ce problème, certains fabricants proposent des substrats dans lesquels le groupement fluorophore est relié à 2 unités saccharidiques par la position anomérique 1. Un exemple de ce type de substrat est la FDG. En multipliant par 2 le nombre d'unités saccharidiques liées au groupement fluorophore, on augmente effectivement l'inhibition initiale de la fluorescence. Néanmoins, la libération du groupement fluorophore et donc de la fluorescence nécessite alors également deux coupures enzymatiques au lieu d'une seule et la sensibilité de la détection n'est donc que peu améliorée dans un tel système.

Il existe également des substrats oligosaccharidiques dont les deux extrémités sont fonctionnalisées par un fluorophore et un inhibiteur de fluorescence ou par deux groupements fluorophores (Cottaz S. et al., Eur. J. Biochem., 2000, 267, 5593-5600). Ces substrats fonctionnent sur le principe représenté sur le schéma B ci-après : 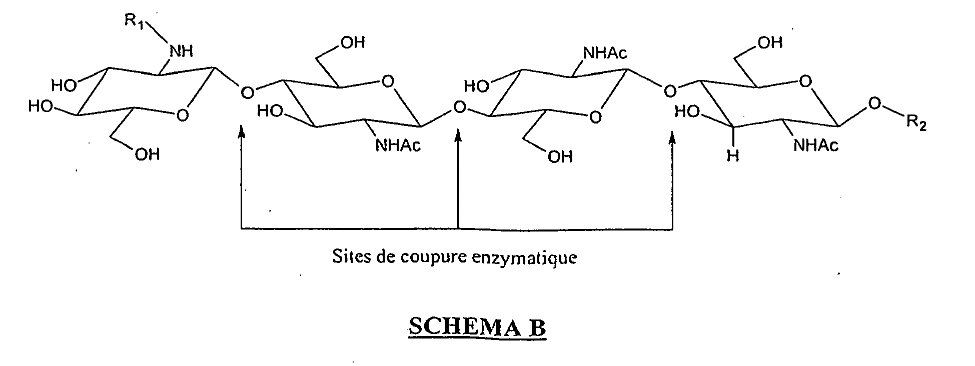 dans lequel R₁ et R₂ représentent respectivement un groupement fluorophore et un inhibiteur de la fluorescence, ou bien deux groupements fluorophores.

Cependant, de tels substrats ne donnent pas entièrement satisfaction dans la mesure où l'inhibition initiale de la fluorescence n'est pas toujours totale et ils mettent en oeuvre des groupements fluorophores ne permettant pas de réaliser une détection *in vivo*.

Le seul substrat enzymatique absorbant et émettant dans le proche infrarouge est le substrat DDAOG qui est un conjugué de β-galactoside (G) et de 7-hydroxy-9*H-*(1,3-dichloro-9,9-diméthylacridin-2-one) (DDAO) utilisé pour la détection de l'activité β-gal et vendu par la société Molecular Probes (USA). Ce substrat absorbe à 645 nm et émet à 660 nm. Son mode de fonctionnement, tel que décrit par Tung C.-H. et al., Cancer Research, 2004, 64, 1579-1583, est représenté sur le Schéma C ci-après :

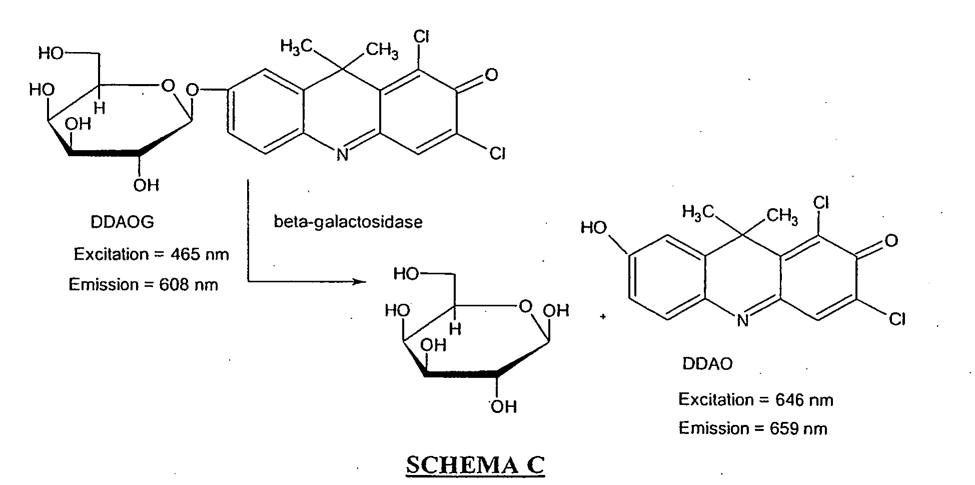

Cependant, bien qu'absorbant et émettant dans le proche infrarouge, ce substrat particulier présente lui aussi un certain nombre d'inconvénients :
- sa fluorescence, lorsque le groupement fluorophore est lié à l'unité saccharidique, n'est pas totalement inhibée, ce qui donne un bruit de fond initialement non négligeable et diminue la sensibilité de détection. Ainsi, on peut constater dans l'expérience *in vivo* relatée dans l'article de Tung C.-H. *et al.,* (précité) que la dose de DDAOG injectée (0,5 mg) et le temps d'exposition nécessaire (2 minutes) sont très importants par rapports aux quantités et temps d'exposition classiques pour ce genre d'applications (en général 10-50 µg de substrat injecté pour un temps d'exposition de 20 à 100 ms) ;
- les spectres d'absorption et d'émission du DDAO sont très étroits et très proches l'un de l'autre, ce qui nécessite un très bon filtrage optique pour la détection du signal par rapport au bruit de fond initial ;
- les spectres d'absorption et d'émission du DDAO ne sont pas encore assez décalés dans le rouge pour être dans une fenêtre optique optimale pour faire de l'imagerie *in vivo.*

C'est donc afin de remédier à l'ensemble de ces problèmes que les Inventeurs ont mis au point ce qui fait l'objet de l'invention.

Les Inventeurs se sont en effet fixés pour but de pourvoir à un substrat enzymatique fluorescent de nature saccharidique n'ayant pas les inconvénients des substrats actuellement disponibles et qui soit en particulier utilisable pour détecter des activités enzymatiques *in vivo*.

La présente invention a donc pour objet un substrat enzymatique fluorescent, caractérisé par le fait qu'il répond à la structure (1) suivante : 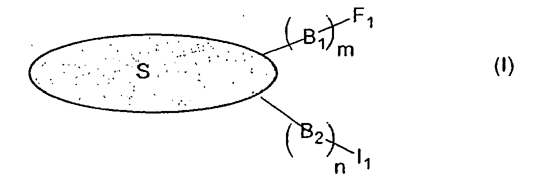 dans laquelle :
- S est un squelette de nature saccharidique constitué d'au moins une unité saccharidique et choisi parmi les monosaccharides, les oligosaccharides ayant de 2 à 9 unités saccharidiques et les polysaccharides ayant au moins 10 unités saccharidiques ;
- B₁ et B₂, identiques ou différents, représentent un bras espaceur constitué d'une chaîne hydrocarbonée linéaire ou ramifiée, saturée ou insaturée, éventuellement substituée, interrompue et/ou terminée par un ou plusieurs hétéroatomes choisis parmi N, O ou S, et/ou par un ou plusieurs groupements choisis parmi les radicaux alkyle en C₁-C₄, alcoxy en C₁-C₄, aryle ou par une ou plusieurs fonctions choisies parmi les fonctions éther, ester, amide, carbonyle, carbamate, urée, thiourée et disulfure ;
- m et n, indépendamment l'un de l'autre, sont des nombres entiers égaux à 0 ou 1 ;
- F₁ est un groupement fluorophore ;
- I₁ est un inhibiteur de la fluorescence de F₁ ; étant entendu que :
   i) F₁ et I₁, soit directement soit par l'intermédiaire des bras espaceurs B₁ et/ou B₂ lorsque m et/ou n = 1, sont tous les deux greffés sur la même unité saccharidique du squelette S ;
   ii) l'un des groupements F₁ et I₁ est greffé en position 1 anomérique de ladite unité saccharidique, la liaison anomérique étant indifféremment en position α ou β, l'autre groupement F₁ ou I₁ occupant n'importe quelle position libre de la même unité saccharidique ; et
   iii) qu'aucune chaîne phosphate, unité saccharidique ou base azotée ne vient s'intercaler entre l'unité saccharidique du squelette S portant les groupements F₁ et I₁ et lesdits groupement F₁ et I₁.

La définition donnée ci-dessus pour B₁ et B₂ exclut de fait tous les bras espaceurs qui comporteraient (ou seraient constitués d') une chaîne phosphate, une unité saccharidique ou une base azotée.

Les substrats enzymatiques conformes à l'Invention et tels que décrits ci-dessus présentent une meilleure inhibition initiale de la fluorescence compte tenu de la proximité spatiale des groupements F₁ et I₁ puisque ceux-ci sont tous les deux greffés sur la même unité saccharidique. Les avantages apportés par les substrats enzymatiques de l'Invention reposent sur le fait que la fluorescence du groupement fluorophore dans le substrat initial (avant action d'une enzyme) n'est pas inhibée par le squelette S mais principalement par l'inhibiteur I₁ présent sur la même unité saccharidique que le groupement fluorophore F₁. Cette configuration particulière permet, par rapport aux substrats connus dans l'état de la technique :
- d'élargir le choix des groupements fluorophores utilisables : le choix d'un tel groupement n'est plus limité aux groupements dont la fluorescence doit initialement être inhibée par le squelette S. Le choix des groupements fluorophores disponibles est donc beaucoup plus large et peut notamment s'étendre aux groupements fluorophores émettant dans le proche infrarouge ;
- d'accroître la sensibilité de détection de l'activité enzymatique : en effet, la fluorescence du groupement fluorophore est inhibée par un objet dont c'est spécifiquement le rôle et qui est ainsi beaucoup plus efficace que lorsque l'inhibition doit être réalisée par le squelette saccharidique ;
- d'accroître la solubilité du substrat par le biais de l'utilisation de groupements fluorophores beaucoup plus solubles que les groupements fluorophores actuellement disponibles dans le commerce et qui sont généralement hydrophobes ;
- de faciliter les conditions d'utilisation du substrat, par le biais de groupements fluorophores insensibles aux variations de pH ou au potentiel RedOx, donc plus facilement utilisables *in vivo*. En effet, certains groupements fluorophores utilisés classiquement, tels que la fluorescéine, ont des propriétés d'émission très dépendantes du pH et donc difficilement compatibles avec une application *in vivo* où les conditions intracellulaires ne peuvent être modifiées. En utilisant des groupements fluorophores dont les propriétés d'émission sont peu dépendantes du pH, du potentiel RedOx ou de la concentration en ions, on facilite les conditions d'acquisition du signal et la sensibilité *in vivo.*

Les unités saccharidiques du squelette S des substrats de structure (I) conformes à l'Invention, peuvent notamment être choisies parmi le galactose, le mannose, l'idose, le talose, le rhamnose, le glucose, le ribose, le fucose et leurs dérivés aminés ou acides parmi lesquels on peut notamment citer la galactosamine, la glucosamine, la lactosamine, l'acide glucuronique, l'acide iduronique et l'acide sialique. Elles sont de préférence choisies parmi la glucosamine, le galactose et l'acide glucuronique.

Lorsque le squelette S est un monosaccharide, ces unités saccharidiques sont utilisées unitairement. Elles sont par contre reliées entre elles par des liaisons glycosidiques lorsque le squelette S est un oligosaccharide ou un polysaccharide.

Selon l'Invention, lorsque le squelette S est un oligosaccharide, il est de préférence choisi parmi les oligosaccharides comportant de 4 à 9 unités saccharidiques.

De façon optionnelle, c'est-à-dire lorsque m et/ou n = 1, les groupements F₁ et/ou I₁ sont reliés à l'unité saccharidique par l'intermédiaire d'un bras espaceur afin de favoriser l'interaction entre le substrat et son enzyme ou l'inhibition de la fluorescence.

Des exemples de bras espaceurs B₁ préférés sont des bras bifonctionnels, tels que l'une de leurs deux extrémités est une fonction réactive vis-à-vis des groupements classiques d'activation de la position anomérique de l'unité saccharidique sur laquelle ils doivent être fixés (exemples notables de groupes d'activation de la position anomérique : -Br, -SPh avec Ph = phényle ; exemple notable de fonction réactives vis-à-vis de ces groupements d'activation : -OH en particulier), et leur autre extrémité est une fonction (par exemple amine ou thiol) réactive vis-à-vis d'une fonction de greffage portée par le groupement fluorophore F₁ ou le groupement inhibiteur I₁ (telle que par exemple une fonction N-hydroxysuccinimidyle, isothiocyanate, ester de sulfotétrafluorophényl (STP-ester), maléimide ou haloacétamide).

Selon une forme de réalisation particulière de l'Invention, et lorsque les groupements F₁ et/ou I₁ sont directement reliés à l'unité saccharidique terminale du squelette S, (m et/ou n = 0) alors le groupement F₁ ou I₁ se trouvant en position 1 anomérique de l'unité saccharidique est relié à cette dernière par l'intermédiaire d'une liaison covalente faisant intervenir au moins un atome X choisi parmi les atomes d'oxygène, de carbone, de souffre et d'azote.

Lorsque m = 0 (respectivement n = 0), le groupement F₁ (respectivement I₁) n'occupant pas la position anomérique peut également être relié à l'unité saccharidique du squelette S par l'intermédiaire d'une liaison covalente faisant intervenir au moins un atome X choisi parmi les atomes d'oxygène, de carbone, de souffre et d'azote.

Ainsi, les groupements F₁ et/ou I₁ peuvent par exemple être reliés à l'unité saccharidique (en position anomérique ou non) par l'intermédiaire d'une liaison amide, ester, thioéther ou thioester, cette énumération n'étant pas exhaustive. Les positions libres des unités saccharidiques du squelette S, qui ne comportent ni le groupement fluorophore F₁, ni le groupement inhibiteur I₁, et qui ne sont pas engagées dans une liaison glycosidique, peuvent indifféremment être non substituées (-H ou -OH) ou bien être substituées par exemple par une fonction amine ou par un groupement résultant de l'interaction d'une fonction hydroxyle ou d'une fonction amine avec un groupement protecteur tel que ceux classiquement utilisés en chimie organique et décrits par exemple dans l'ouvrage de T. W. Greene et al., "Protective Groups in Organic Synthesis", Third Edition, Wiley Science (1999). Parmi de tels groupements protecteurs, on peut notamment citer les groupements acétyle ; benzyle ; aryle et en particulier les groupements aryle, substitués par un radical choisi parmi les chaînes alkyle ayant de 1 à 40 atomes de carbone ; 2,2,2-trichloroéthyloxycarbonyle (Troc) ; benzyloxycarbonyle (BzC) ; trichloroacétamidate (TCA) ; tert-butyloxycarbonyle (BOC), fluoranylméthoxycarbonyle (Fmoc), ainsi que les groupements silylés tels que par exemple les groupements t-butyldiméthylsilyle (tBDMS) et triméthylsilyle (TMS).

Parmi les groupements fluorophores F₁, on peut notamment citer la fluorescéine (fluorescéinate de sodium) et ses dérivés tels que l'isothiocyanate de fluorescéine (FITC) ; les colorants fluorescents absorbant et émettant dans le proche infrarouge ("Near InfraRed" : NIR) tels que ceux vendus sous les dénominations Fluorescent Red NIR 700 (longueur d'onde d'excitation : 672 nm ; longueur d'émission : 735 nm) et Fluorescent Red NIR 730 (longueur d'onde d'excitation : 680 nm ; longueur d'émission : 755 nm) par la société Sigma-Aldrich ; le Cy5 (n=2) et le Cy7 (n = 3) (Amersham) ; le 7-hydroxy-9*H-*(1,3-dichloro-9,9-diméthylacridin-2-one) (DDAO), la rhodamine et ses dérivés tels que la tetraméthyl rhodamine isothiocyanate (TRITC) ; les colorants fluorescents à amines réactives telles que les coumarines parmi lesquelles on peut notamment citer l'ester succinimidylique de l'acide 6-((7-amino-4-méthylcoumarin-3-acétyl)amino) hexanoïque (AMCA) ; les colorants fluorescents vendus sous les dénominations commerciales BODIPY ® tels que BODIPY® FR-Br₂, BODIPY ® R6G, BODIPY® TMR, BODIPY ® TR et les BODIPY ® 530/550 (longueur d'onde d'excitation/longueur d'onde d'émission, en nm), 558/568, 564/570, 576/589, 581/591, 630/650 et 650/665 vendus par la société Bio-Rad Inc. (USA), IRDye ® 800 vendu par la société LICOR et Alexa Fluor ® 750 et Alexa Fluor ® 633 vendus par la société Molecular Probes ; les porphyrines ; les cyanines ; les oxazines et les nanoparticules fluorescentes c'est-à-dire ayant des propriétés d'émission telles que les "quantum dots", les nanoparticules d'or, les nanoparticules à base de polymères et les nanoparticules d'oxydés.

Selon une forme de réalisation particulièrement préférée de l'Invention, le groupement F₁ est choisi parmi les groupements fluorophores absorbant et émettant dans le proche infrarouge, c'est-à-dire émettant et absorbant à une longueur d'onde comprise entre 640 et 900 nm. Parmi de tels groupements, on peut en particulier citer les groupements fluorophores suivants : les colorants fluorescents vendus sous les dénominations Fluorescent Red NIR 700 (longueur d'onde d'excitation (Ex.) : 672 nm / longueur d'émission (Em.) : 735 nm) et Fluorescent Red NIR 730 (Ex : 680 nm / Em : 755 nm) par la société Sigma-Aldrich ; le Cy5 (n = 2 ; Ex. : 680 nm / Em. : 755 nm) et le Cy7 (n = 3 : Ex. : 747 nm / Em. : 775 nm) (Amersham) ; le 7-hydroxy-9*H-*(1,3-dichloro-9,9-diméthylacridin-2-one) (DDAO) (Ex. : 646 nm / Em. : 659 nm), l'lRDye ® 800 (Ex. : 778 / EM. : 806 nm), l'Alexa Fluor ® 750 (Ex. : 749 nm / Em. : 774 nm), l'Alexa Fluor ® 633 (Ex. : 633 nm / Em : 647 nm). Selon une forme de réalisation particulière de l'invention, le groupement fluorophore F₁ peut en outre être fonctionnalisé par un ou plusieurs groupements choisis parmi les chaînes lipophiles, les phospholipides et les peptides.
Ainsi fonctionnalisés, les groupements fluorophores F₁ présentent une plus grande affinité pour les cellules des tissus animaux.

Selon l'Invention, le groupement I₁ peut être choisi parmi tous les composés acceptant la fluorescence du groupement F₁, c'est à dire permettant la diminution ou la disparition complète de la fluorescence du groupement F₁ lorsqu'ils sont tous deux fixés à la même unité saccharidique du squelette S des substrats de structure (I). Ce composé, de natures diverses, peut notamment être un groupement chimique, fluorescent ou non fluorescent, ou une nanoparticule.

Lorsque le groupement I₁ est lui-même un groupement fluorescent, alors il est choisi parmi les groupements dont la fluorescence inhibe celle du groupement F₁ (on parle alors d'auto-inhibition, la fluorescence du groupement I₁ inhibant celle du groupement F₁. Dans ce cas, le groupement I₁ est généralement identique au groupement F₁ et est choisi parmi les cyanines, les groupements fluorophores Cy5, Cy7, l'IRDye ® 800, l'Alexa Fluor ® 750 et l'Alexa Fluor ® 633.
A titre de groupement I₁, on peut également utiliser un groupement fluorescent, différent du groupement F₁, qui absorbe la fluorescence du groupement F₁ par transfert d'énergie par résonance de fluorescence (FRET). Dans ce cas on préfère utiliser les couples F₁/I₁ suivants : Cy5/Cy7 ; Cy5/Alexa Fluor ® 750 ; Alexa Fluor ® 633/Cy7; Alexa Fluor ® 633/Alexa Fluor ® 750 ; Cy7/]RDye ® 800 ; Alexa Fluor ® 750/IRDye ® 800.

Lorsque le groupement I₁ est un groupement non-fluorescent, c'est à dire un inhibiteur de fluorescence proprement dit ("Quencher"), alors il est de préférence choisi parmi les composés vendus sous les dénominations commerciales DABCYL ® et dérivés, Black Hole Quencher ® (BHQ) telles que BHQ 1, BHQ 2 ou BHQ 3 (Biosearch Technologies), Nanogold Particules ® (Nanoprobes), Eclipse Dark Quencher ® (Epoch Bioscience), Elle Quencher ® (Oswell), Cy7Q (Amersham), FluoQuench ® tels que FluoQuench ® 660 et FluoQuench ® 661 (FluoProbes), et les colorants QSY ® tels que les QSY ® 7, QSY ® 9 et QSY ® 21 (Molecular Probes).

Selon l'Invention, les groupements F₁ et I₁ sont utilisés tels qu'ils se présentent commercialement, ces groupements pouvant notamment comporter, outre la partie chromophore proprement dite, un groupement fonctionnel de greffage tel que par exemple une fonction N-succinimidyle, maléimide, acide carboxylique, amine, etc... et éventuellement un bras espaceur situé entre ladite fonction de greffage et ladite partie chromophore. Les bras espaceurs B₁ et B₂ éventuellement présents dans les composés de formule (I) conformes à l'Invention sont alors d'autres bras espaceurs que l'on incorpore entre le groupement de greffage des groupements F₁ et I₁ et l'unité saccharidique du squelette S.

Aussi, suivant la nature du groupement I₁, et dans l'hypothèse où les groupements F₁ et I₁ sont tous les deux reliés à l'unité saccharidique terminale du squelette S par l'intermédiaire d'un bras espaceur B₁ et B₂ respectivement, le principe de fonctionnement des substrats enzymatiques de structure (I) conformes à l'invention peut être représenté par le Schéma D suivant : 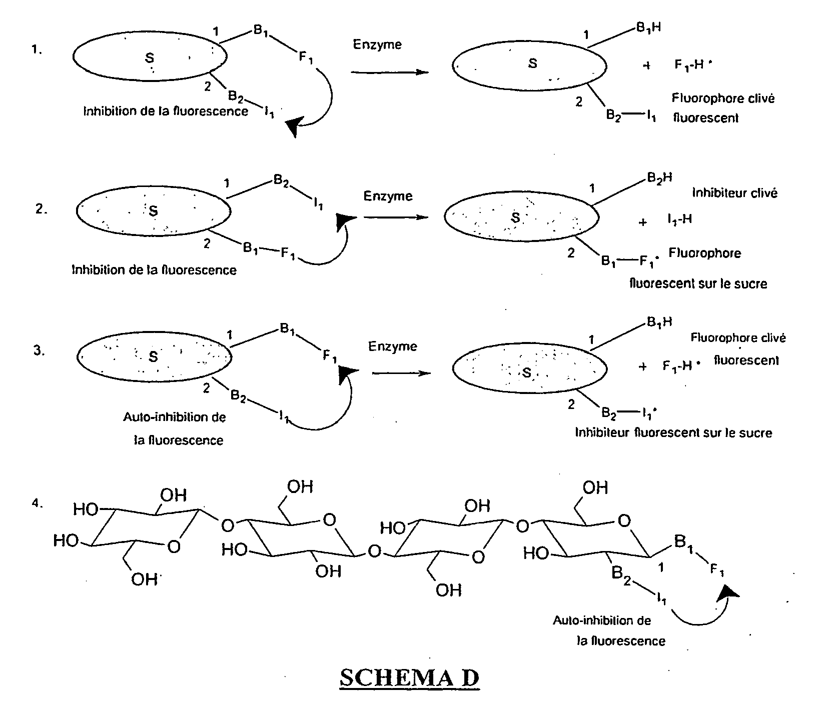 dans lequel les groupements B₁ et B₂, identiques ou différents les uns des autres, peuvent prendre l'une des significations indiquées précédemment. Selon ce schéma :
1. Configuration 1 : le groupement F₁ est fixé en position 1 anomérique d'une unité saccharidique d'un squelette S par l'intermédiaire d'un bras espaceur B₁, le groupement I₁ est un inhibiteur de la fluorescence de type "quencher" fixé dans une autre position de la même unité saccharidique du squelette S par l'intermédiaire d'un bras espaceur B₂, dans le cas présent en position 2. Le clivage enzymatique libère alors le fluorophore F₁ du squelette S. Eloigné de l'inhibiteur I₁, le groupement fluorophore F₁ émet.
2. Configuration 2 : le groupement I₁ de type "quencher" est fixé en position 1 anomérique d'une unité saccharidique d'un squelette S par l'intermédiaire d'un bras espaceur B₂, le groupement F₁ fluorophore est fixé dans une autre position de la même unité saccharidique du squelette S par l'intermédiaire d'un bras espaceur B₁, dans le cas présent, en position 2. Le clivage enzymatique libère le groupement I₁ du squelette S. Eloigné de l'inhibiteur, le groupement fluorophore F₁ qui reste fixé sur le squelette sucre émet. L'avantage d'une telle configuration est que le groupement F₁ reste fixé sur le squelette saccharidique qui *a priori* reste localisé dans la cellule.
3. Configuration 3: le groupement F₁ est un groupement fluorophore fixé en position 1 anomérique d'une unité saccharidique d'un squelette S par l'intermédiaire d'un bras espaceur B₁ ; le groupement I₁ est un groupement fluorescent dont la fluorescence inhibe la fluorescence du groupement F₁, fixé dans une autre position de la même unité saccharidique du squelette S par l'intermédiaire d'un bras espaceur B₂, dans le cas présent en position 2. Le clivage enzymatique libère le groupement F₁ qui se retrouve éloigné de I₁. F₁ n'est plus inhibé par I₁. Les deux groupements F₁ et I₁ émettent. L'avantage d'une telle structure est que la fluorescence observée est double. Cette configuration convient particulièrement bien à la détection d'activités enzymatiques de faible intensité si F₁ est identique à I₁.
4. Configuration 4 : l'unité saccharidique du squelette S comportant les groupements F₁ et I₁ est reliée, par une liaison glycosidique à au moins une autre unité saccharidique, pour former par exemple un tetrasaccharide.

Panni les substrats enzymatiques de structure (I) conformes à l'Invention, on préfère tout particulièrement les composés dans lesquels :
i) le squelette S est une galactosamine, F₁ et I₁ sont identiques et sont choisis parmi les groupements Cy5, Alexa Fluor ® 750, Cy7 et IRDye ® 800, ces groupements étant respectivement fixés en positions 1 et 2 de la galactosamine ;
ii) le squelette S est une galactosamine, F₁ est un groupement Cy5, Alexa Fluor ® 750, Alexa Fluor ® 633 ou IRDye ® 800 et I₁ est un groupement QSY ® 21, Cy7Q ou BHQ3 ;
iii) le squelette S est une lactosamine, F₁ est un groupement Cy5, Cy7, Alexa Fluor ® 750 ou IRDye ® 800 et I₁ est un groupement QSY ® 21, Cy7Q ou BHQ 3 ;
iv) le squelette S est un trisaccharide comportant une glucosamine en position terminale et dont les deux autres unités saccharidiques, identiques ou différente, sont choisies parmi le galactose et le fucose, F₁ est un groupement Cy5, Cy7, Alexa Fluor ® 750 ou IRDye ® 800 et I₁ est un groupement QSY ® 21, Cy7Q ou BHQ 3.

Les substrats enzymatiques de formule (I) conformes à l'Invention
dans lesquels les groupements F₁ et I₁ sont différents l'un de l'autre peuvent par exemple être préparés selon un procédé (P1) comportant au moins les étapes suivantes :
i) dans une première étape, on fait subir au squelette S, dont la position anomérique 1 est déprotégée et la position sur laquelle on souhaite greffer la seconde fonctionnalité (F₁ ou I₁) comporte une fonction réactive faisant intervenir au moins un atome X' (X' = N, O ou S) protégée par un groupement protecteur R tels que ceux cités précédemment (T. W. Greene *et al.,* 1999, précité), une réaction d'activation de la position anomérique (greffage d'un groupement R' en position 1, R' représentant une fonction réactive (R' représente par exemple -Br ou -SPh avec Ph = phényle) ;
ii) dans une deuxième étape; on fait réagir le produit obtenu à l'étape (i), dont la position anomérique est activée par R' :
   - soit avec un groupement fluorophore F₁ (respectivement avec un inhibiteur I₁) comportant une fonction terminale (T, avec T = -OH par exemple) réactive vis-à-vis du groupement R' en position anomérique,
   - soit, dans un premier temps, avec un bras espaceur bifonctionnel comportant à l'une de ses extrémités une fonction terminale (T) réactive vis-à-vis de la fonction réactive R' en position anomérique et à l'autre extrémité une fonction (R", avec R" = amine ou thiol par exemple) réactive vis-à-vis d'une fonction de greffage (G, avec G = N-hydroxysuccinimidyle, isothiocyanate, ester de sulfotétrafluorophényl (STP-ester), maléimide ou haloacétamide par exemple) portée par le groupement fluorophore (respectivement inhibiteur), qui dans un deuxième temps réagit avec ladite fonction réactive R" du bras espaceur ;
iii) dans une troisième étape, on effectue une déprotection de la fonction -XR présente sur la position sur laquelle on souhaite greffer la seconde fonctionnalité par clivage du groupement protecteur R, pour obtenir un composé comportant une fonction déprotégée -XH,
iv) dans une quatrième étape, le produit obtenu ci-dessus à l'étape iii) est mis à réagir :
   - soit avec un groupement inhibiteur I₁, différent de F₁ (respectivement un groupement fluorophore F₁, différent de I₁) comportant une fonction terminale (T) réactive vis-à-vis de la fonction -XH,
   - soit dans un premier temps, avec un bras espaceur bifonctionnel comportant à l'une de ses extrémités une fonction terminale (T) réactive vis-à-vis de la fonction -XH et à l'autre extrémité une fonction (R") réactive vis-à-vis d'une fonction de greffage (G) portée par le groupement inhibiteur (respectivement fluorophore), qui dans un deuxième temps réagit avec ladite fonction réactive R" du bras espaceur, pour obtenir le composé de structure (I) correspondant.

Ce procédé P1 est représenté sur le schéma E ci-après :

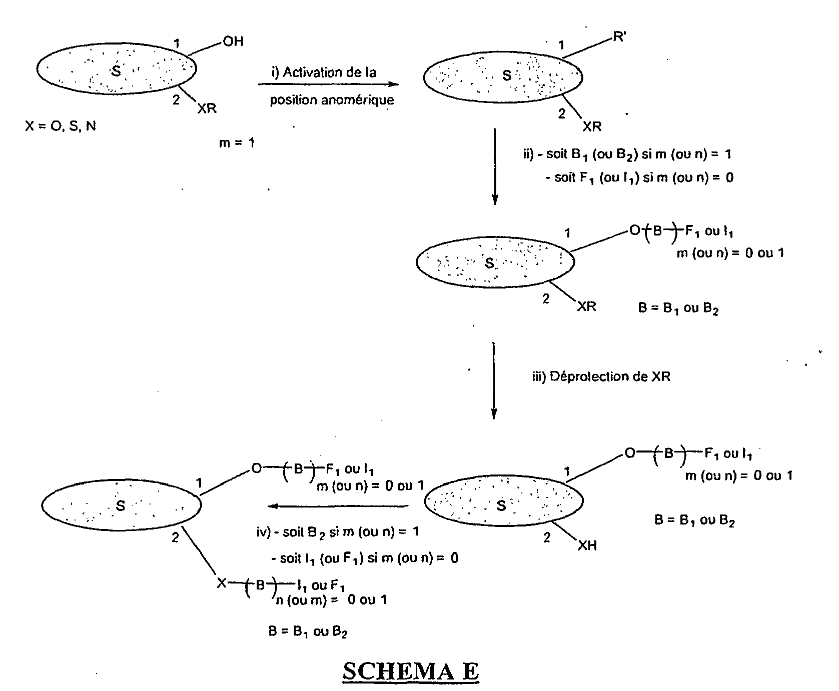

Le procédé P1 représenté sur le Schéma E ci-dessus comprend un nombre minimal d'étapes. Il doit toutefois être bien entendu que suivant la nature du substrat enzymatique de formule (I) que l'on souhaite obtenir, des réactions supplémentaires de protection/déprotection peuvent être nécessaires. Ces réactions sont réalisées de façon classique, selon les méthodes connues de l'homme du métier.

Selon ce procédé, il doit également être compris que les bras espaceurs B₁ et B₂, lorsque tous les deux présents, peuvent être identiques ou différents l'un de l'autre.

Par ailleurs, bien que ce procédé de synthèse P1 soit particulièrement bien adapté à la synthèse de substrats enzymatiques de formule (1)
dans lesquels les groupements F₁ et I₁ sont différents l'un de l'autre, il est néanmoins parfaitement utilisable également pour la préparation de substrats enzymatiques de formule (I) dans lesquels les groupements F₁ et I₁ seraient identiques, notamment dans le cas où ces groupements seraient fixés à l'unité saccharidique par l'intermédiaire de bras espaceurs B₁ et B₂ avec B₁ différent de B₂.

Les substrats enzymatiques de formule **(I)** conformes à l'Invention
dans lesquels les groupements F₁ et I₁ sont identiques peuvent également être préparés par exemple selon un procédé P2 comportant au moins les étapes suivantes :
i) dans une première étape, on fait subir au squelette S, dont la position anomérique 1 est déprotégée et la position sur laquelle on souhaite greffer la seconde fonctionnalité (F₁ ou I₁) comporte une fonction faisant intervenir au moins un atome X' (X' = N, O ou S) protégée par un groupement protecteur R tels que ceux cités précédemment, une réaction d'activation de la position anomérique (greffage d'un groupement R' en position 1, R' représentant une fonction réactive (R' représente par exemple -Br ou -SPh avec Ph = phényle) ;
ii) dans une deuxième étape, on effectue une déprotection de la fonction -XR présente sur la position sur laquelle on souhaite greffer la seconde fonctionnalité par clivage du groupement protecteur R, pour obtenir un composé comportant une fonction déprotégée -XH,
iii) dans une troisième étape, on fait réagir le produit obtenu à l'étape (ii), dont la position anomérique est activée par R' et qui comporte une fonction -XH déprotégée :
   - soit avec un groupement fluorophore F₁ (F₁ = I₁) comportant une fonction terminale (T, avec T = -OH par exemple) réactive vis-à-vis du groupement R' en position anomérique et de la fonction déprotégée -XH, pour obtenir un composé bifonctionalisé par un groupement F₁ (avec F₁ = I₁),
   - soit, dans un premier temps, avec un bras espaceur bifonctionnel comportant à l'une de ses extrémités une fonction terminale (T) réactive vis-à-vis de la fonction réactive R' en position anomérique et de la fonction déprotégée -XH et à l'autre extrémité une fonction (R", avec R" = amine ou thiol par exemple) réactive vis-à-vis d'une fonction de greffage G telle que définie précédemment, portée par le groupement fluorophore (F₁ = I₁), qui dans un deuxième temps réagit avec ladite fonction réactive R" du bras espaceur ;
      pour obtenir un substrat enzymatique de formule (I) dans lequel F₁ = I₁ et dans lequel, lorsqu'ils sont présents, B₁ = B₂.

Ce procédé P2 est donc particulièrement bien adapté au cas où les substrats enzymatiques comportent des groupements fluorophore F₁ et inhibiteur I₁ identiques l'un par rapport à l'autre puisqu'ils se trouvent greffés sur l'unité saccharidique simultanément, éventuellement par l'intermédiaire d'un bras espaceur, également identique d'un groupement à l'autre. Ce procédé P2 est représenté sur le Schéma F ci-après :

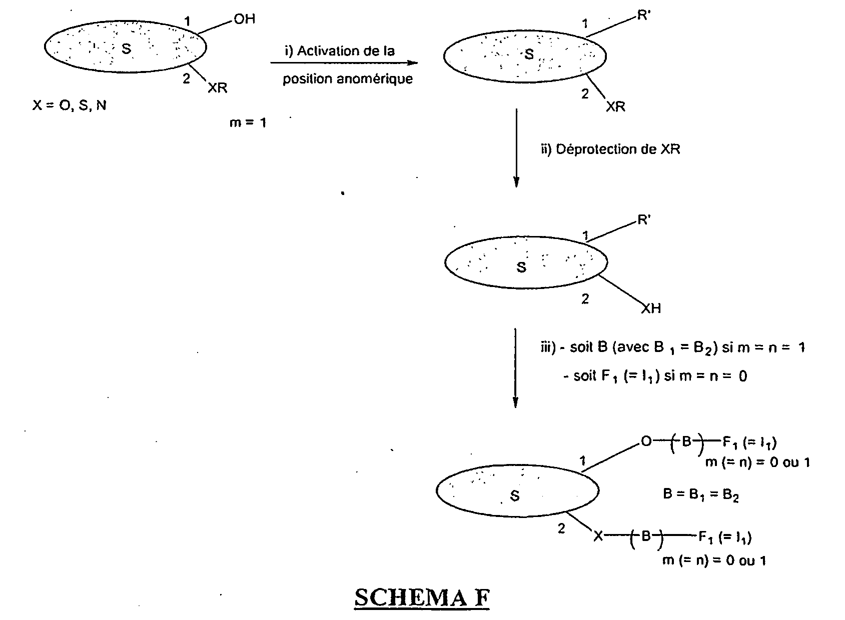

Selon une variante de l'Invention, ce procédé P2 peut également être utilisé pour la préparation de substrats enzymatiques de formule (1) dans lesquels les groupements F₁ et I₁ ne sont pas forcément identiques l'un par rapport à l'autre. Dans ce cas, l'étape iii) est réalisée en présence d'un mélange de F₁ et I₁ (avec F₁ différent de I₁) et conduit à l'obtention d'une bibliothèque de 4 substrats enzymatiques de formule (I) doublement marqués et différents les uns des autres :
- Substrat enzymatique (Ia) : squelette S dont l'unité saccharidique terminale est doublement marquée par un groupement F₁ (un en position anomérique et l'autre sur une autre position de la même unité saccharidique) ;
- Substrat enzymatique (Ib) : squelette S dont l'unité saccharidique terminale est doublement marquée par un groupement I₁ (un en position anomérique et l'autre sur une autre position de la même unité saccharidique) ;
- Substrat enzymatique (Ic) : squelette S dont l'unité saccharidique terminale est marquée par un groupement F₁ en position anomérique et par un groupement I₁ sur une autre position de la même unité saccharidique ;
- Substrat enzymatique (Id) : squelette S dont l'unité saccharidique terminale est marquée par un groupement I₁ en position anomérique et par un groupement F₁ sur une autre position de la même unité saccharidique.

Ces substrats enzymatiques (Ia) à (Id) peuvent être séparés les uns des autres par les techniques classiques séparatives bien connues de l'homme du métier telles que la chromatographie liquide haute performance (HPLC), la chromatographie sur silice, la séparation de charges. Après séparation, ces substrats peuvent être purifiés et étudiés de façon indépendante.

Dans le cas particulier où les groupements F₁ et I₁ portent en plus des charges opposées (par exemple F₁ = Cy5 chargé négativement et I₁ = QSY ® 21 chargé positivement), on peut alors bénéficier des avantages suivants :
- la séparation des quatre substrats enzymatiques est facilitée. On obtient en effet un substrat enzymatique de type (Ia) chargé négativement (F₁ = I₁ = Cy5), un substrat enzymatique de type (Ib) chargé positivement (F₁ = I₁ = QSY® 21) et 2 substrats enzymatiques de type (Ic) et (Id) qui sont neutres (Cy5 en position anomérique et QSY® 21 sur une autre position et QSY® 21 en position anomérique et Cy5 sur une autre position Ainsi, les substrats enzymatiques chargés peuvent être séparés des substrats enzymatiques neutres par séparation des charges par chromatographie sur gel. Les substrats enzymatiques neutres peuvent être séparés entre eux par HPLC ou par chromatographie sur colonne de silice, bien qu'il soit également possible d'utiliser le mélange des deux substrats enzymatiques tel quel ;
- les deux substrats enzymatiques peuvent présenter des avantages pour la détection d'activité enzymatiques *in vivo.* En effet, une molécule neutre pénétrera mieux dans le milieu intracellulaire. En revanche, la dégradation enzymatique des ces substrats conduira dans ce cas à deux molécules chargées qui seront mieux retenues dans le milieu intracellulaire qu'une molécule neutre.

Selon une variante du procédé P2, l'étape iii) peut également être réalisée en utilisant un mélange contenant plusieurs groupements F₁ (pF₁ avec p = nombre de groupements F₁ différents) et plusieurs groupements I₁ (qI₁ avec q = nombre de groupements I₁ différents), dans une approche de chimie combinatoire. On obtient alors statistiquement (p+q)² substrats enzymatiques de formule (I) différents qui peuvent ensuite être séparés (par exemple par purification HPLC, chromatographie sur silice, séparation de charges). Après séparation, ces différents substrats enzymatiques peuvent être purifiés et étudiés indépendamment les uns des autres. On obtient ainsi une bibliothèque de (p+q)² substrats enzymatiques de formule (I) dont le potentiel en imagerie *in vivo* peut ensuite être évalué via un "screening".

A chaque étape de ces procédés P1 et P2, les composés intermédiaires, et le(s) composé(s) final (finaux) de structure (1) en fin de synthèse, sont de préférence lavés, isolés et purifiés selon les méthodes classiquement utilisées à cet effet telles que par exemple la purification sur colonne.

A titre d'exemple, et lorsque le squelette S des composés de formule (I) est un monosaccharide tel que la galactosamine, n = 0, m = 1 avec B₁ = -CH₂-CH₂-NH et F₁ et I₁ sont tous deux des groupements fluorescents, identiques ou différents l'un de l'autre, on peut également synthétiser les composés conformes à invention en utilisant le schéma général de synthèse G suivant :

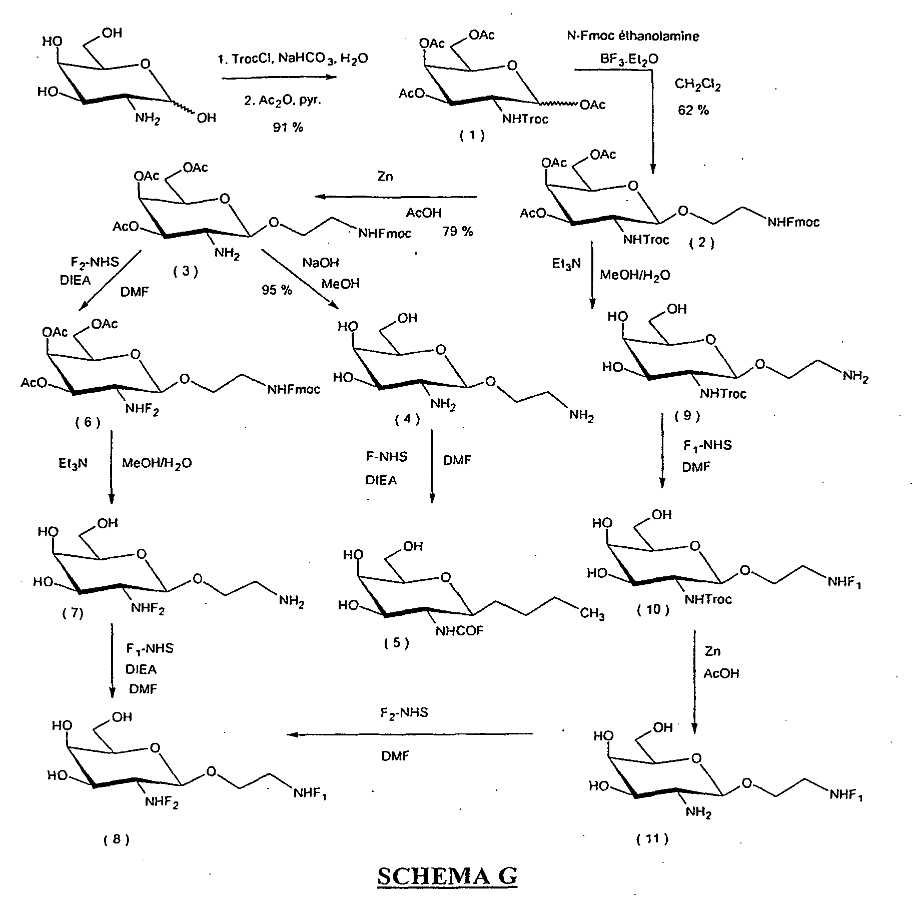

Sur ce schéma, les abréviations utilisées ont la signification suivante :
- TrocCl : chlorure de 2,2,2-trichloroéthyloxycarbonyle,
- NaHCO₃ : hydrogénocarbonate de sodium,
- Ac₂O : anhydride acétique,
- pyr : pyridine,
- F : groupement fluorescent (F, F1 ou F₂),
- N-Fmoc éthanolamine : N-α- fluoranylméthoxycarbonyle-éthanolamine,
- BF₃.Et₂O : éthérate de trifluorure de bore,
- CH₂Cl₂ : dichlorométhane (DCM),
- Zn : zinc,
- AcOH : acide acétique,
- DIEA : diisopropyléthylamine,
- DMF : diméthylformamide,
- NaOH : soude,
- MeOH : méthanol,
- ET₃N : triéthylamine.

Ainsi que cela a été amplement décrit et explicité ci-avant, les substrats enzymatiques de structure (I) conformes à l'Invention peuvent être utilisés pour la détection d'une activité enzymatique *in vitro* et *in vivo.*

La présente Invention a donc pour deuxième objet, l'utilisation d'au moins un substrat enzymatique de structure (I) tel que défini précédemment, à titre de réactif fluorescent pour la détection d'une activité enzymatique *in vitro.*

Selon une forme de réalisation particulièrement préférée, le groupement fluorophore F₁ des substrats enzymatiques de structure (I) est choisi parmi les groupements absorbant et émettant dans le proche infrarouge, en particulier entre 640 et 900 nm, afin de permettre une utilisation *in vivo.* Dans ce cas, la présente Invention a également pour objet, l'utilisation d'au moins un substrat enzymatique de structure (I) dans lequel le groupement fluorophore F₁ est choisi parmi les groupements absorbant et émettant dans le proche infrarouge, pour la préparation d'un réactif de diagnostic destiné à l'imagerie fonctionnelle *in vivo* et en particulier pour imager, par fluorescence, l'expression des gènes rapporteurs *lacZ* et *gusA* de *E. coli.*

Enfin l'Invention a pour objet un réactif de diagnostic caractérisé par le fait qu'il comprend au moins une solution constituée d'eau ou d'un mélange d'eau et d'au moins un solvant organique, ladite solution renfermant au moins un substrat enzymatique de structure (I) tel que défini précédemment.

Selon une forme de réalisation particulière et préférée de l'invention, le réactif est un réactif de diagnostic *in vivo* et le substrat enzymatique de structure (1) comprend au moins un groupement fluorophore F₁ choisi parmi les groupements fluorophores absorbant et émettant dans le proche infrarouge.

A titre de solvants organiques utilisables, on peut citer les solvants classiquement utilisés pour la préparation de réactifs de diagnostic parmi lesquels figurent par exemple les alcools inférieurs tels que l'éthanol et le diméthylsulfoxyde (DMSO). Lorsque qu'ils sont utilisés, ces solvants peuvent représenter jusqu'à 50 % (en volume) de la solution renfermant le substrat enzymatique de structure (1).

Selon une forme de réalisation particulière de l'Invention, la solution peut en outre renfermer un tampon physiologiquement acceptable tel qu'un tampon phosphate comme par exemple du PBS ("Phosphate Buffer Saline") à pH 7,2.

Au sein du réactif de diagnostic conforme à l'Invention, la concentration du ou des substrats enzymatiques de structure (I) est de préférence comprise entre 1 µM et 1 mM environ, plus préférentiellement entre 10 µM et 200 µM environ. Selon une forme de réalisation particulièrement préférée de l'Invention, cette concentration est de 100 µM environ.

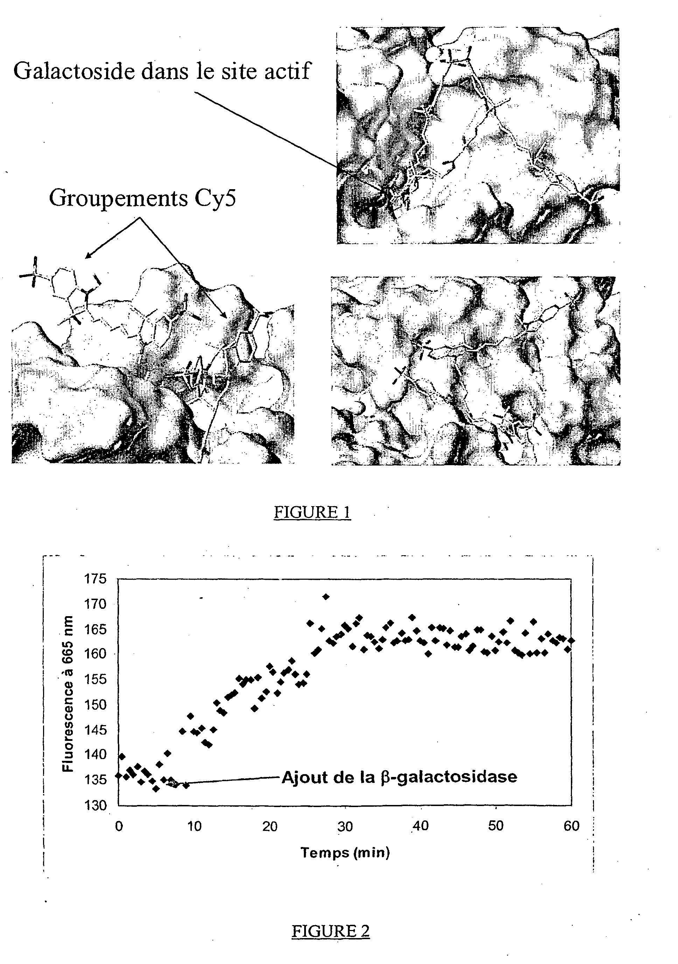

Outre les dispositions qui précèdent, l'Invention comprend encore d'autres dispositions qui ressortiront de la description qui va suivre, qui se réfère à des exemples de préparation et d'utilisation de substrats saccharidiques fluorescents de formule (I) conforme à l'Invention, ainsi qu'aux figures 1 à 6 annexées dans lesquelles :
- la figure 1 représente la modélisation moléculaire d'un complexe entre la β-galactosidase et un galactoside greffé par deux groupements Cy5 à l'aide du logiciel de modélisation vendu sous la dénomination commerciale Sybyl® 7.0 par la société TRIPOS Inc.
- la figure 2 représente la fluorescence (unités arbitraires) mesurée à 665 nm en fonction du temps en minutes, lors de l'ajout de β-galactosidase (15 UI) dans une solution du composé de formule **(5a)** à 1 µM, dans 1,5 ml de tampon phosphate (PBS) à pH 7,4 et à une température de 20°C ;

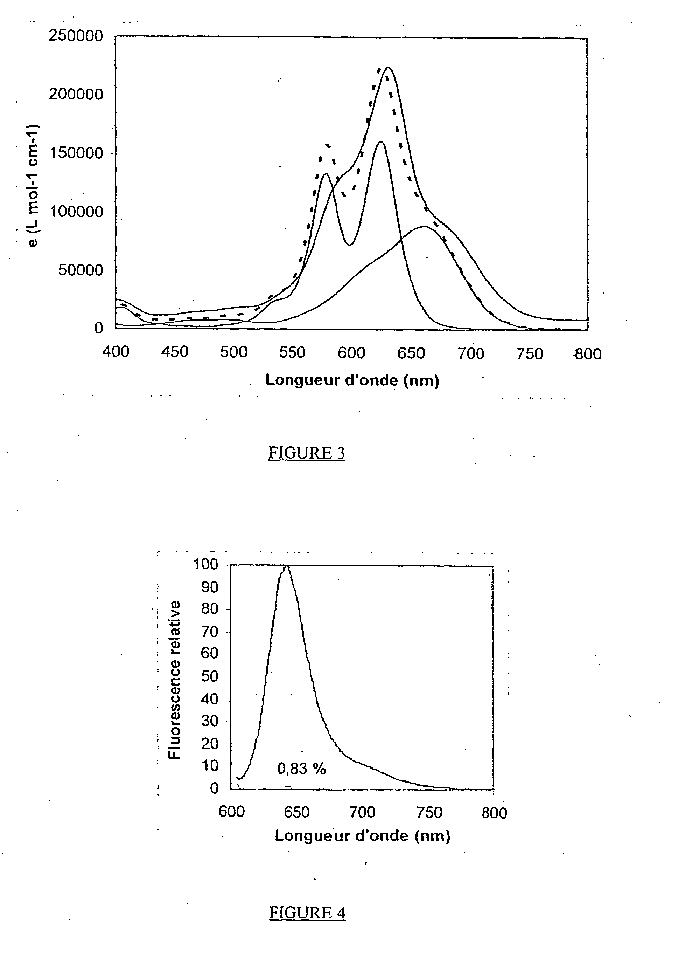

- la figure 3 représente les spectres d'absorption du composé de formule **(8a)** en solution à 3,5 µM dans du tampon phosphate 10 mM pH 7,2 (PBS) (courbe pleine la plus haute), comparativement à ceux de l'Alexa Fluor ® 633 seul en solution à 3,5 µM dans le PBS (courbe pleine intermédiaire), du QSY® 21 seul en solution à 3,5 µM dans le méthanol (courbe pleine la plus basse) et du mélange QSY ® 21 à 3,5 µM/Alexa Fluor ® 633 à 3,5 µM en solution dans le PBS (courbe en pointillés). Sur cette figure l'absorption e (en L mol⁻¹ cm⁻¹) est exprimée en fonction de la longueur d'onde (nm) ; .
- la figure 4 représente les spectres d'émission (après excitation à 600 nm) du composé de formule **(8a)** (courbe la plus basse, pratiquement inexistante) comparativement à l'Alexa Fluor ® 633 (courbe la plus haute). Sur cette figure la fluorescence (unités arbitraires) est exprimée en fonction de la longueur d'onde (nm) ;

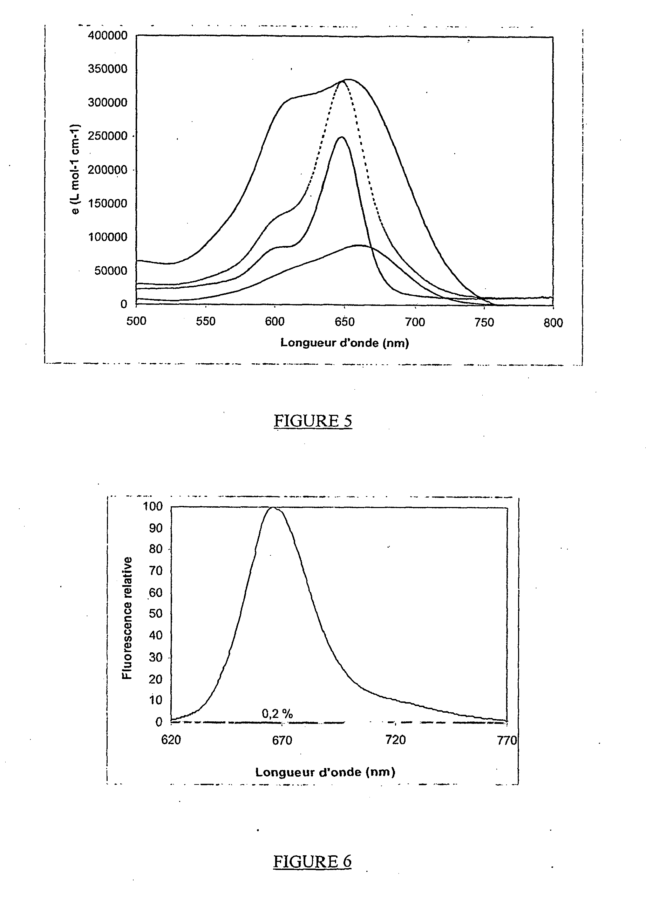

- la figure 5 représente le spectre d'absorption du composé de formule **(8b)** en solution à 0,99 µM dans le PBS (courbe continue la plus haute), comparativement à ceux de du QSY® 21 seul en solution à 0,99 µM dans le méthanol (courbe continue la plus basse), du Cy5 seul en solution à 0,99 µM dans le PBS (courbe continue intermédiaire) et du mélange QSY ® 21 à 0,99 µM/Cy5 à 0,99 µM en solution dans le PBS (courbe en pointillés). Sur cette figure l'absorption e (en L mol⁻¹ cm⁻¹) est exprimée en fonction de la longueur d'onde (nm) ;
- la figure 6 représente les spectres d'émission (après excitation à 600 nm) du composé de formule **(8b)** (courbe inexistante) comparativement au Cy5 (courbe haute). Sur cette figure la fluorescence (unités arbitraires) est exprimée en fonction de la longueur d'onde (nm).

### EXEMPLE 1 : SYNTHESE ET UTILISATION DU COMPOSE DE FORMULE (5a) : Gal(N-Cy5)-Cy5

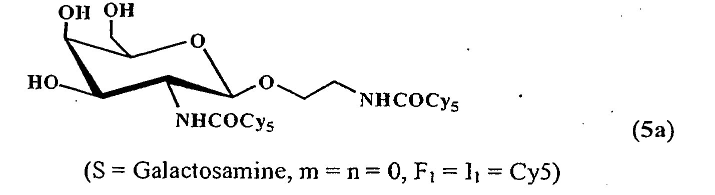

### I) Synthèse

### I) Première Etape : Synthèse de la 1,3,4,6-tétra-O-acétyl-2-désoxy-2-(2,2,2-trichloroéthoxycarbonylamino)-β-D-galactopyranose (1)

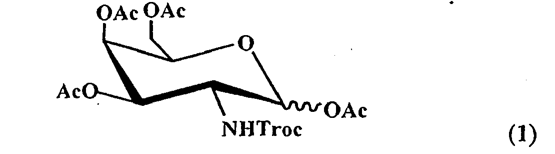

A une solution de 0,5 g de galactosamine (2,32 mmole) dans 6 ml d'eau distillée ont été additionnés, goutte à goutte, 0,65 g (3 éq.) d'hydrogénocarbonate de sodium et 0,43 ml (1,2 éq.) de chlorure de trichloroéthoxycarbonyle. Le milieu réactionnel a été agité pendant 1 heure puis le solvant a été évaporé sous vide. Le résidu obtenu a alors été dissous dans 15 ml de pyridine et 2 ml (8 éq.) d'anhydride acétique ont alors été ajoutés. Le milieu réactionnel a été agité pendant 15 heures puis le solvant a été évaporé sous pression réduite. Le brut a été chromatographié sur gel de silice (éluant : Cyclobexane/acétate d'éthyle (AcOEt) : 3/2, v/v) pour donner 11, g (2,11 mmoles) du composé **(1)** attendu avec un rendement de 91 %.

L'analyse structurale du produit obtenu était conforme à celle du produit attendu :
¹H RMN (CDCl₃) : δ = 6,30 (d, 1H, NHTroc, J_{NHTroc,2} = 3,5 Hz) ; 5,46 (m, 1H, H-4) ; 5,23 (m, 2H, H-1 et H-3) ; 4,76 (s, 2H, COO-CH₂-CCl₃) , 4,48 (m, 1H, H-2) ; 4,42 (dd, 1H, H-6a, J_{6a-6b} = 11,2 Hz, J_{6a,5} = 4,0 Hz) ; 4,28 (m, 1H, H-5) ; 4,23 (dd, 1H, H-6b, J6b,5 = 4,5 Hz) ; 2,22 (s, 3H, CH₃CO); 2,15 (s, 3H, CH₃CO); 2,07 (s, 3H, CH₃CO) ; 2,11 (s, 3H, CH₃CO).
¹³C RMN (CDCl₃) : δ = 170,7 ; 170,6 ; 169,3 ; 149,6 ; 95,7 ; 91,5 ; 68,9 ; 68,2 ; 67,0 ; 61,6 ; 21,0
ESI : m/z = 523 [M+H]⁺

### 2) Deuxième Etape : Synthèse de la 2-hydroxy-N-9-fluorénylméthoxycarbonyl-1-aminoéthyl-3,4,6-tri-O-acétyl-2-désoxy-2-(2,2,2-trichloroéthoxycarbonylamino)-β-D-galactopyranose (2)

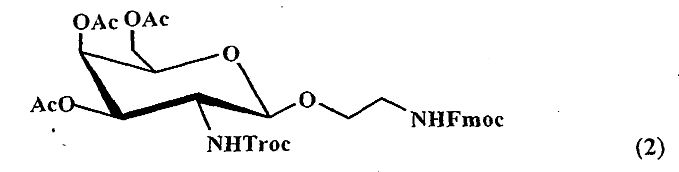

0,6 g de (1,15 éq.) de 1,3,4,6-tétra-*O*-acétyl-2-désoxy-2-(2,2,2-trichloroéthoxycarbonylamino)-β-D-galactopyranose **(1)** obtenu ci-dessus à l'étape 1) a été dissous dans 15 ml de dichlorométhane anhydre puis on a ajouté 400 mg (1,2 éq.) de *N*-Fmoc éthanolamine et 0,45 ml (3 éq.) de trifluoroéthérate de bore (BF₃.Et₂O). Le mélange réactionnel a été agité pendant 5 heures, dilué dans du dichlorométhane (DCM) puis lavé par une solution de HCl (1M). Les phases organiques ont été rassemblées, séchées et concentrées sous pression réduite. Le brut obtenu a alors été purifié par chromatographie sur gel de silice (éluant Cyclohexane/AcOEt 2/1 : v/v). Le composé (**2**) (0,53 g ; 0,71 mmole) a alors été isolé avec un rendement de 62%.

L'analyse structurale du produit obtenu était conforme à celle du produit attendu :
¹H RMN (CDCl₃) : δ = 7,78 (m, 2H, Ha) ; 7,61 (m, 2H, Hd) ; 7,35 (m, 4H, Hc et Hd) ; 5,44 (m, 1H, NHFmoc) ; 5,30 (d, 1H, H-4, J_{4,3} = 3,5 Hz) ; 5,23 (d, 1H, H-1, J_{1,2} = 8,9 Hz) ; 5,12 (dd, 1H, H=3, J_{3,2} = 11,1 Hz) ; 4,70 (m, 3H, H-5 et COOCH₂CCl₃); 4,39 (m, 2H, NHTroc et H-6a) ; 4,21 (m, 1H, H-6b) ; 4,03 (d, 2H, Hf, J_{f,e} = 6,8 Hz) ; 3,90 (dd, 1H, H-2, J_{2,3} = 11,1 Hz) ; 3,76 (m, 2H, O-CH₂-CH₂-N); 3,53 (t, 1H, He) ; 3,36 (m, 2H, O-CH₂-CH₂-N) ; 2,15 (s, 3H, CH₃CO) ; 2,00 (s, 3H, CH₃CO); 1,90 (s, 3H, CH₃CO).
¹³C RMN (CDCl₃ : δ = 170,7 ; 171,0 ; 157,1 ; 155,0 ; 144,2 ; 141,7 ; 128,2 ; 127,6 ; 125,3 ; 120,4 ; 102,0 ; 96,0 ; 74,7 ; 71,0 ; 70,3 ; 67,0 ; 66,7 ; 66,3 ; 62,2 ; 53,0 ; 47,7 ; 41,4 ; 27,3 ; 21,1.
ESI : m/z = 745 [M+H]⁺

### 3) Troisième Etape : Synthèse du 2-hydroxy-N-9-fluorénylméthoxycarbonyl-1-aminoéthyl-3,4,6-tri-O-acétyl-2-désoxy-2-amino-β-D-galactopyranose (3)

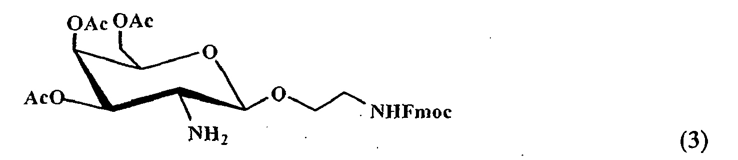

A 0,2 g (0,34 mmole) de 2-hydroxy-*N*-9-fluorénylméthoxycarbonyl-1-aminoéthyl-3,4,6-tri-*O*-acétyl-2-désoxy-2-(2,2,2-trichloroéthoxycarbonylamino)-β-D-galactopyranose (**2**) obtenu ci-dessus à l'étape 2), solubilisé dans 4 ml d'acide acétique, on a additionné 0,15 g de zinc activé. Le mélange réactionnel a été agité pendant 6 heures puis le zinc a été éliminé par filtration sur célite. Le filtrat a été concentré sous pression réduite et le produit cristallisé dans l'éther éthylique. On a obtenu alors 0,12 g (0,27 mmole) de composé (**3**) avec un rendement de 79 %.

L'analyse structurale du produit obtenu était conforme à celle du produit attendu :
¹H RMN (CDCl₃) : δ = 7,74 (m, 2H, Ha) ; 7,58 (m, 2H, Hd) ; 7,34 (m, 4H, Hc et Hd) ; 5,85 (m, 1H, NHFmoc) ; 5,36 (d, 1H, H-4, J_{4,3} = 3,3 Hz) ; 5,25 (d, 1H, H-1, J_{1,2} = 9,2 Hz) ; 4,86 (m, 2H, H-3 et H-5) ; 4,20 (m, 2H, H-6) ; 4,03 (d, 2H, Hf, J_{f,e} = 6,8 Hz) ; 4,00 (m, 2H, O-CH₂-CH₂-N) ; 3,90 (dd, 1 H, H-2) ; 3,53 (m, 1H, He) ; 3,40 (m, 2H, O-CH₂-CH₂-N) ; 2,07 (s, 3H, CH₃CO); 2,04 (s, 3H, CH₃CO) ; 1,87 (s, 3H, CH₃CO).
¹³C RMN (CDCl₃) : δ = 170,6 ; 170,4 ; 170,9 ; 144,1 ; 141,5 ; 127,9 ; 127,4; 125,2; 120,2 ; 100,6 ; 71,0 ; 69,8 ; 68,2 ; 66,5 ; 61,8 ; 52,9; 47,3 ; 41,3 ; 21,3 ; 20,8 ; 20,7.
ESI : m/z = 571 [M+H]⁺

### 4) Quatrième Etape : Synthèse du 1-aminoéthyl-2-amino-2-désoxy-β-D-galactopyranose (4)

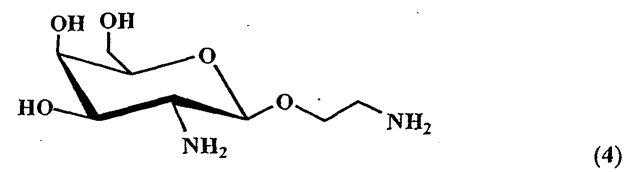

0,1 g (0,17 mmole) de 2-hydroxy-*N*-9-fluorénylméthoxycarbonyl-1-aminoéthyl-3,4,6-tri-*O*-acétyl-2-désoxy-2-amino-β-D-galactopyranose (**3**) tel qu'obtenu ci-dessus à l'étape 3) a été dissous dans 5 ml de méthanol puis on a ajouté 13,6 ml (8 éq.) d'une solution de soude 1N. Le mélange réactionnel a été agité pendant 1 heure puis neutralisé par addition d'une résine échangeuse de cation vendue sous la dénomination Dowex 50WX8 par la société Sigma-Aldrich. La résine a ensuite été éliminée par filtration sur verre fritté et le filtrat a été concentré sous pression réduite. 0,32 g (0,14 mmoles) de composé (**4**) a alors été isolé par cristallisation dans du DCM avec un rendement de 84 %.

L'analyse structurale du produit obtenu était conforme à celle du produit attendu :
¹H RMN (D₂O) : δ = 4,19 (d, 1H, H-1, J_{1,2} = 8,1 Hz) ; 3,85 (m, 1H, H-4) ; 3,68 (dd, 1H, H-3, J_{3,4} = 4,7 Hz ; J_{3,2} = 11,9 Hz) ; 3,58 (m, 3H, H-5 et O-CH₂-CH₂-N) ; 3,49 (m, 4H, H-6 et O-CH₂-CH₂-N).; 3,68 (dd, 1H, H-2)
¹³C RMN (D₂O) : δ = 103,2 ; 75,5 ; 73,0 ; 69,2 ; 68,1 ; 61,2 ; 53,2 ; 40,0.
ESI : m/z = 223 [M+H]⁺

### 5) Cinquième Etape : Obtention du composé de formule (5a), Gal(N-Cy5)-Cy5

On a additionné 0,13 mg (5,85.10⁻⁴ mmoles) de 1-aminoéthyl-2-amino-2-désoxy-β-D-galactopyranose (4) tel qu'obtenu ci-dessus à l'étape 4) et 50 µl de triéthylamine à une solution de Cy5NHS (1 mg ; 1,14.10⁻³ mmole) dissous dans 200 µl de diméthylsulfoxyde (DMSO). Le milieu réactionnel a été agité pendant 15 heures puis concentré sous pression réduite. Le composé (**5a**) a alors été obtenu après purification par chromatographie liquide haute performance (HPLC).

L'analyse structurale du produit obtenu était conforme à celle du produit attendu :
ESI : m/z = 1581 [M+H]⁺

### II) Utilisation du composé de formule (5a) pour la détection de l'activité enzymatique de la β-galactosidase

### II - 1) Détection de l'activité enzymatique par modélisation moléculaire

Des modélisations moléculaires des interactions entre la β-galactosidase (β-gal) et le composé de formule (**5a**) le Gal(N-Cy5)-(Cy5), ont été réalisées afin de déterminer les potentialités de ce produit comme substrat pour cette enzyme.

Les données utilisées pour la modélisation de l'enzyme sont issues de l'étude de D. Juers *et al.* (Code PDB 1JYV, Juers DH, Heightman TD, Vasella A, et al. "A structural view of the action of Escherichia coli (lacZ) β-galactosidase.", Biochemistry, 2001, 40, 14781-14794.) donnant les coordonnées cristallographiques de β-gal en interaction avec un substrat colorimétrique : l'Ortho Nitro Phényl Galactopyranoside (ONPG). La molécule de galactose fonctionnalisée par deux groupements Cy5 a été construite dans le logiciel de modélisation vendu sous la dénomination commerciale Sybyl® 7.0 par la société TRIPOS Inc. en utilisant l'éditeur de molécules disponible, et en ajoutant les groupements chimiques à un galactose provenant de la banque de données tridimensionnelles du CERMAV (http://www.cermay.cnrs.fr/glyco3d). Les deux groupements Cy5 ont été construits
- dans des conformations étendues. Cette molécule a ensuite été placée dans le site actif de l'enzyme à la place du substrat d'origine, l'ONPG.

Les résultats obtenus sont représentés sur la figure 1 annexée,

Ils montrent que les deux bras des groupements Cy5 en conformation étendue ne génèrent que très peu de conflit stérique avec la protéine. La géométrie du complexe a donc pu être directement optimisée de façon à obtenir une configuration de basse énergie (les charges de la protéine ont été attribuées d'après la librairie de charges Kollman, celles attribuées au substrat sont issues de la librairie Gasteiger-Huckel). Seules les chaînes latérales des acides aminés proches du site de liaison et la molécule de Cy5 ont été optimisées. La configuration représentée sur la figure 1 possède une énergie suffisamment basse pour que l'on puisse penser que la formation de ce complexe est possible. En effet, même si le site actif de l'enzyme est relativement profond, la longueur des chaînes alkyles (de 6 à 9 atomes de carbone) reliant le galactose aux deux groupements Cy5 est suffisante pour permettre à ces groupements cyanines de se situer à la surface de la protéine.

Ainsi il est possible de penser que le composé (**5a**) conforme à la présente invention pourra bien être un substrat pour l'enzyme β-gal.

### II-2) Utilisation du composé (5a) pour la détection de l'activité enzymatique de la β-galactosidase : test in vitro

L'activité enzymatique de la β-galactosidase vis-à-vis du composé de formule (**5a**) conforme à l'Invention : le Gal(N-Cy5)-(Cy5), a également été testée *in vitro*.

Le principe de fonctionnement des nouveaux substrats enzymatiques de formule (I) conformes à l'Invention est représenté sur le schéma H ci-après :

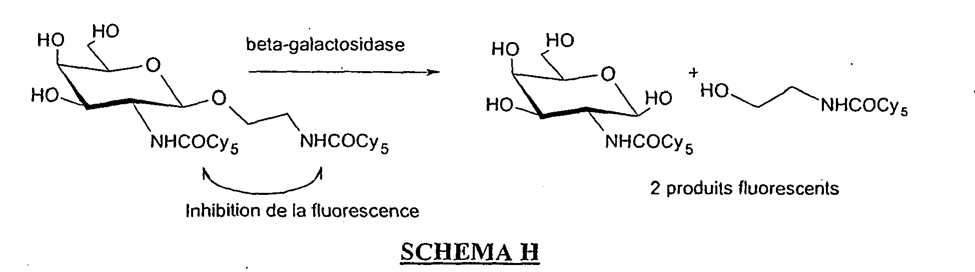

La proximité spatiale entre deux Cy5 conduit à une inhibition initiale de leur fluorescence. La fluorescence est recouvrée après coupure enzymatique du substrat.

On a par ailleurs préparé une solution du composé de formule (**5a**) à 1 µM, dans 1,5 ml de tampon phosphate (PBS) à pH 7,4 à laquelle on a ajouté 15 unités internationales (UI) de β-galactosidase. La fluorescence a ensuite été mesurée à une longueur d'onde de 665 nm, en fonction de la durée d'incubation à 20°C, à l'aide d'un fluorimètre vendu sous la dénomination commerciale LS50B par la société Perkin Elmer.

Les résultats obtenus sont représentés sur la figure 2 annexée sur laquelle la fluorescence mesurée en unités arbitraires est exprimée en fonction du temps en minutes.

Sur cette figure, on constate une augmentation de la fluorescence (+ 25%) lors de l'ajout de la β-galactosidase dans la solution de composé de formule (**5a**) avec une constante cinétique du pseudo-1^{er} ordre de 17 minutes qui confirme les prévisions obtenus par modélisation moléculaire.

### EXEMPLE 2 : SYNTHESE ET PROPRIETES DU COMPOSE DE FORMULE (8a) : Gal(N-OSY® 21)-Alexa Fluor® 633

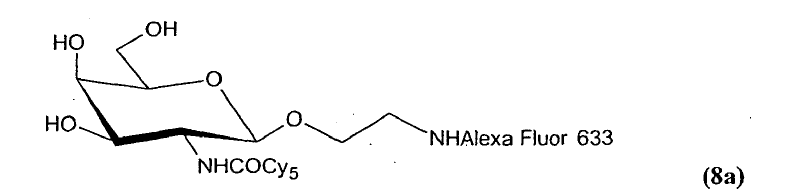

(S = galactosamine, m = n = 0, F₁ = Alexa Fluor ® 633, I₁ = QSY® 21)

### I) Synthèse

Cette synthèse se fait à partir du composé intermédiaire de formule (**3**) dont la préparation a été décrite précédemment à l'étape 3) de l'exemple 1.

### 1) Première Etape : Synthèse du composé intermédiaire de formule (6a) : Gal(N-QSY® 21)-NHFmoc peracétylée

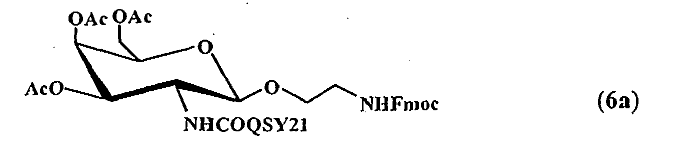

A une solution de 0,7 mg (1,22.10⁻³ mmoles) de 2-hydroxy-*N*-9-fluorénylméthoxycarbonyl-1-aminoéthyl-3,4,6-tri-*O*-acétyl-2-désoxy-2-amino-β-D-galactopyranose (3) tel qu'obtenu ci-dessus à l'étape 3) de l'exemple 1, solubilisé dans 70 µl de DMF anhydre, on a additionné 1 mg de QSY® 21NHS (1 éq.) préalablement dissous dans 100 µl de DMF anhydre. On a ensuite ajouté 50 µl de diisopropyléthylamine (DIEA), puis le mélange réactionnel a été agité pendant 15 heures. Le solvant a ensuite été évaporé sous pression réduite et le composé (**6a**) a été obtenu après purification par HPLC.

L'analyse structurale du produit obtenu était conforme à celle du produit attendu :
ESI : m/z = 1235 [M+H]⁺

### 2) Deuxième Etape : Synthèse du composé intermédiaire de formule (7a) : Gal(N-QSY® 21)- NH₂

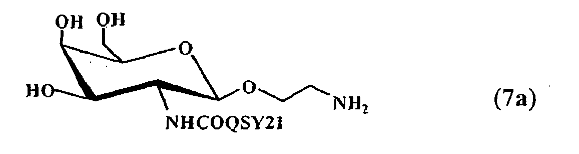

La Gal(N-QSY® 21)-NHFmoc peracétylée de formule (**6a**) a été solubilisée dans 600 µl d'un mélange méthanol/eau/triéthylamine (MeOH/H₂O/TEA : 4/1/1). Le milieu réactionnel a été agité pendant 15 heures puis concentré sous pression réduite. Le composé (**7a**) a alors été obtenu après purification par HPLC.

L'analyse structurale du produit obtenu était conforme à celle du produit attendu :
ESI : m/z = 887 [M+H]⁺

### 3) Troisième Etape : Obtention du composé de formule (8a) : Gal(N-OSY® 21)-Alexa Fluor ® 633

A une solution de 1,1 mg (1,14.10⁻³ mmoles) de Gal(N-QSY® 21)-NH₂ de formule (**7a**) obtenu ci-dessus à l'étape précédente, solubilisés dans 100 µl de DMF anhydre on a additionné 1 mg (1 éq.) d'Alexa Fluor ® 633NHS préalablement dissous dans 100 µl de DMF anhydre et 50 µl de DIEA. Le mélange réactionnel a été agité pendant 15 heures puis concentré sous pression réduite. Le composé de formule (**8a**) a alors été obtenu après purification par HPLC.

L'analyse structurale du produit obtenu était conforme à celle du produit attendu :
ESI : m/z = 930 [M+3H]⁺/2

### II) Propriétés photophysiques du composé de formule (8a)

Le spectre d'absorption IR du composé de formule (**8a**) a été déterminé en solution à 3,5 µM dans le PBS (courbe pleine la plus haute), comparativement à ceux de l'Alexa Fluor ® 633 seul en solution à 3,5 µM dans le PBS (courbe pleine intermédiaire), du QSY® 21 seul en solution à 3,5 µM dans le méthanol (courbe pleine la plus basse) et du mélange QSY ® 21 à 3,5 µM/Alexa Fluor ® 633 à 3,5 µM en solution dans le PBS (courbe en pointillés), à l'aide d'un appareil vendu sous la dénomination CARY 300 par la société VARAN.

Les spectres correspondants sont reportés sur la figure 3 annexée sur laquelle l'absorption (L mol⁻¹ cm⁻¹) est exprimée en fonction de la longueur d'onde (nm).

Ces spectres confirment qu'il y a bien eu greffage à la fois du fluorophore Alexa Fluor ® 633 et de l'inhibiteur de fluorescence QSY® 21 sur la galactosamine.

Les spectres d'émission du composé de formule (**8a**) et de l'Alexa Fluor ® 633, après excitation à une longueur d'onde de 600 nm, ont également été mesurés et sont représentés sur la figure 4 annexée sur laquelle la fluorescence relative (unités arbitraires) est exprimée en fonction de la longueur d'onde (en nm). Sur cette figure, la courbe haute représente la fluorescence observée pour l'Alexa Fluor ® 633, la courbe basse, pratiquement inexistante représente celle du composé de formule (**8a**).

Ces résultats montrent que la proximité de l'inhibiteur de fluorescence conduit bien à une inhibition de la fluorescence du fluorophore (99,2 % d'inhibition).

### EXEMPLE 3 : SYNTHESE ET PROPRIETES DU COMPOSE DE FORMULE (8a) : Gal(N-Cy5)-QSY® 21

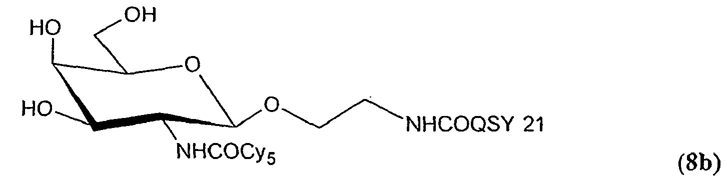

S = galactosamine ; m = n = 0 ; F₁ = QSY® 21 et I₁ = Cy₅

Cette synthèse se fait à partir du composé intermédiaire de formule (3) dont la synthèse a été décrite précédemment à l'étape 3) de l'exemple 1.

### 1) Première Etape : Synthèse du composé intermédiaire de formule (6b) :Gal(N-Cy5)-NHFmoc peracétylée

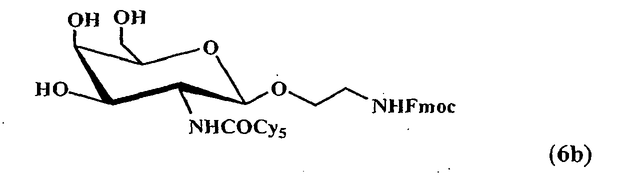

A une solution de 0,7 mg (1,26.10⁻³ mmoles) de 2-hydroxy-*N*-9-fluorénylméthoxycarbonyl-1-aminoéthyl-3,4,6-tri-*O*-acétyl-2-désoxy-2-amino-β-D-galactopyranose de formule (3) tel qu'obtenu ci-dessus à l'étape 3) de l'exemple 1 dans 70 µl de DMF anhydre, on a additionné 1 mg de Cy5NHS (1 éq.) préalablement dissous dans 50 µl de DMF anhydre. On a ajouté ensuite 50 µl de DIEA puis le mélange réactionnel a été agité pendant 15 heures. Le solvant a ensuite été évaporé sous pression réduite et le composé de formule (**6b**) a été obtenu après purification par HPLC.

L'analyse structurale du produit obtenu était conforme à celle du produit attendu :
ESI : m/z = 1248 [M+H]⁺

### 2) Deuxième Etape : Synthèse de l'intermédiaire (7b) : Gal(N-Cy5)-NH₂

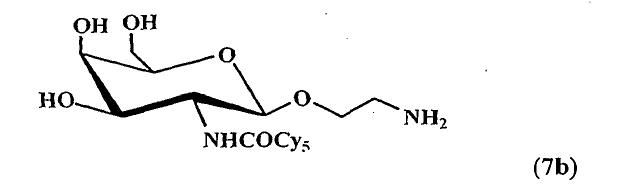

La Gal(N-Cy5)-NHFmoc peracétylée de formule (**6b**) obtenue ci-dessus à la première étape a été solubilisée dans 300 µl d'un mélange MeOH/H₂O/TEA : 4/2/1 (v/v/v). Le milieu réactionnel a été agité pendant 15 heures puis concentré sous pression réduite. Le composé de formule (**7b**) a alors été obtenu après purification par HPLC.

L'analyse structurale du produit obtenu était conforme à celle du produit attendu :
ESI : m/z = 901 [M+H]⁺

### 3) Troisième Etape : Obtention du composé de formule (8b) Gal(N-Cy5)-QSY

A une solution de 1 mg (1,11.10⁻³ mmoles) de Gal(N-Cy5)-NH2 de formule (**7b**) tel qu'obtenu ci-dessus à l'étape 2) précédente, dans 100 µl de DMF anhydre on a additionné 1 mg (1 éq.) de Cy5NHS dissous dans 100 µl de DMF anhydre et 50 µl de DIEA. Le mélange réactionnel a été agité pendant 15 heures puis concentré sous pression réduite. Le composé de formule (**8b**) a alors été obtenu après purification par HPLC.

L'analyse structurale du produit obtenu était conforme à celle du produit attendu :
ESI : m/z = 797,5 [M+H]⁺/2

### II) Propriétés photophysiques du composé de formule (8b)

La figure 5 représente le spectre d'absorption du composé de formule (**8b**) en solution à 0,99 µM dans le PBS (courbe continue la plus haute), comparativement à ceux de du QSY® 21 seul en solution à 0,99 µM dans le méthanol (courbe continue la plus basse), du Cy5 seul en solution à 0,99 µM dans le PBS (courbe continue intermédiaire) et du mélange QSY® 21 à 3,5 µM/Cy5 à 3,5 µM en solution dans le PBS (courbe en pointillés). Sur cette figure l'absorption e (en L mol⁻¹ cm⁻¹) est exprimée en fonction de la longueur d'onde (nm) ;

Ces spectres confirment qu'il y a bien eu greffage à la fois du fluorophore Cy5 et de l'inhibiteur de fluorescence QSY® 21.

Les spectres d'émission du composé de formule (**8b**) et du Cy5, après excitation à une longueur d'onde de 600 nm, ont également été mesurés et sont représentés sur la figure 6 annexée sur laquelle la fluorescence relative (unités arbitraires) est exprimée en fonction de la longueur d'onde (en nm). Sur cette figure, la courbe haute représente la fluorescence observée pour le Cy5, aucune fluorescence n'étant observée pour le composé de formule (**8b**). La proximité de l'inhibiteur de fluorescence conduit bien à une inhibition de la fluorescence du fluorophore (99,8% d'inhibition).

## Revendications

1. Substrat enzymatique fluorescent, **caractérisé par le fait qu'**il répond à la structure (I) suivante : 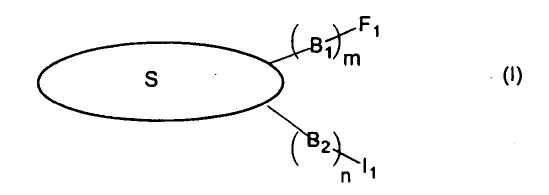 dans laquelle :
- S est un squelette de nature saccharidique constitué d'au moins une unité saccharidique et choisi parmi les monosaccharides, les oligosaccharides ayant de 2 à 9 unités saccharidiques et les polysaccharides ayant au moins 10 unités saccharidiques ;
- B₁ et B₂, identiques ou différents, représentent un bras espaceur constitué d'une chaîne hydrocarbonée linéaire ou ramifiée, saturée ou insaturée, éventuellement substituée, interrompue et/ou terminée par un ou plusieurs hétéroatomes choisis parmi N, O ou S, et/ou par un ou plusieurs groupements choisis parmi les radicaux alkyle en C₁-C₄, alcoxy en C₁-C₄, aryle ou par une ou plusieurs fonctions choisies parmi les fonctions éther, ester, amide, carbonyle, carbamate, urée, thiourée et disulfure ;
- m et n, indépendamment l'un de l'autre, sont des nombres entiers égaux à 0 ou 1 ;
- F₁ est un groupement fluorophore ;
- I₁ est un inhibiteur de la fluorescence de F₁ ; étant entendu que :
i) F₁ et I₁, soit directement, soit par l'intermédiaire des bras espaceurs B₁ et/ou B₂ lorsque m et/ou n = 1, sont tous les deux greffés sur la même unité saccharidique du squelette S ;
ii) l'un des groupements F₁ et I₁ est greffé en position 1 anomérique de ladite unité saccharidique, la liaison anomérique étant indifféremment en position α ou β, l'autre groupement F₁ ou I₁ occupant n'importe quelle position libre de la même unité saccharidique ; et
iii) qu'aucune chaîne phosphate, unité saccharidique ou base azotée ne vient s'intercaler entre l'unité saccharidique du squelette S portant les groupements F₁ et I₁ et lesdits groupement F₁ et I₁.

2. Substrat selon la revendication 1, **caractérisé par le fait que** les unités saccharidiques du squelette S des substrats de structure (I) sont choisies parmi le galactose, le mannose, le idose, le talose, le rhamnose, le glucose, le ribose, le fucose et leurs dérivés aminés ou acides.

3. Substrat selon la revendication 2, **caractérisé par le fait que** les dérivés aminés et acides des unités saccharidiques sont choisis parmi la galactosamine, la glucosamine, la lactosamine, l'acide glucuronique, l'acide iduronique et l'acide sialique.

4. Substrat selon la revendication 2 ou 3, **caractérisé par le fait que** les unités saccharidiques du squelette S des substrats de structure (I) sont choisies parmi la glucosamine, le galactose et l'acide glucuronique.

5. Substrat selon l'une quelconque des revendications précédentes,
**caractérisé par le fait que** le squelette S est un oligosaccharide choisi parmi les oligosaccharides comportant de 4 à 9 unités saccharidiques.

6. Substrat selon l'une quelconque des revendications 1 à 5,
**caractérisé par le fait que** lorsque les groupements F₁ et/ou I₁ sont directement reliés à l'unité saccharidique terminale du squelette S, alors le groupement F₁ ou I₁ se trouvant en position 1 anomérique de l'unité saccharidique est relié à cette dernière par l'intermédiaire d'une liaison covalente faisant intervenir au moins un atome X choisi parmi les atome d'oxygène, de carbone, de souffre et d'azote.

7. Substrat selon l'une quelconque des revendications précédentes,
**caractérisé par le fait que** les positions libres des unités saccharidiques du squelette S, qui ne comportent ni le groupement fluorophore F₁, ni le groupement inhibiteur I₁, et qui ne sont pas engagées dans une liaison glycosidique, peuvent indifféremment être non substituées (-H ou -OH) ou substituées par une fonction amine ou par un groupement résultant de l'interaction d'une fonction hydroxyle ou d'une fonction amine avec un groupement protecteur.

8. Substrat selon l'une quelconque des revendications précédentes,
**caractérisé par le fait que** le groupement fluorophore est choisi parmi la fluorescéine et ses dérivés ; les colorants fluorescents absorbant et émettant à une longueur d'onde comprise entre 640 et 900 nm ; le Cy5 (n = 2) et le Cy7 (n = 3); le 7-hydroxy-9*H-*(1,3-dichloro-9,9-diméthylacridin-2-one), la rhodamine et ses dérivés ; les colorants fluorescents à amines réactives ; les colorants fluorescents vendus sous les dénominations commerciales BODIPY®, IRDye® 800, Alexa Fluor® 750 et Alexa Fluor® 633 ; les porphyrines ; les cyanines ; les oxazines et les nanoparticules fluorescentes.

9. Substrat selon la revendication 8, **caractérisé par le fait que** le groupement fluorophore est choisi parmi les groupements fluorophores absorbant et émettant à une longueur d'onde comprise entre 640 et 900 nm.

10. Substrat selon la revendication 9, **caractérisé par le fait que** le groupement F₁ est choisi parmi les colorants fluorescents vendus sous les dénominations Fluorescent Red NIR 700 et Fluorescent Red NIR 730, le Cy5 (n = 2), le Cy7 (n = 3), le 7-hydroxy-9*H*-(1,3-dichloro-9,9-diméthylacridin-2-one) (DDAO), l'IRDye® 800, l'Alexa Fluor® 750 et l'Alexa Fluor® 633.

11. Substrat selon l'une quelconque des revendications précédentes,
**caractérisé par le fait que** le groupement fluorophore F₁ est en outre fonctionnalisé par un ou plusieurs groupements choisis parmi les chaînes lipophiles, les phospholipides et les peptides.

12. Substrat selon l'une quelconque des revendications précédentes,
**caractérisé par le fait que** le groupement I₁ est un groupement fluorescent qui inhibe
ou diminue ou provoque la disparition complète de la fluorescence du groupement F₁.

13. Substrat selon la revendication 12, **caractérisé par le fait que** le groupement I₁ inhibe la fluorescence de F₁, est identique au groupement F₁ et est choisi parmi les cyanines, les groupements fluorophores Cy5, Cy7, l'IRDye® 800, l'Alexa Fluor® 750 et l'Alexa Fluor® 633.

14. Substrat fluorescent selon la revendication 12, **caractérisé par le fait que** le groupement I₁ est un groupement fluorescent, différent du groupement F₁, et qui absorbe à la longueur d'onde à laquelle le groupement F₁ émet une fluorescence, par transfert d'énergie par résonance de fluorescence.

15. Substrat fluorescent selon la revendication 14, **caractérisé par le fait que** les couples F₁/I₁ sont choisis parmi les couples Cy5/Cy7 ; Cy5/Alexa Fluor® 750 ; Alexa Fluor® 633/Cy7 ; Alexa Fluor® 633/Alexa Fluor® 750 ; Cy7/IRDye® 800 et Alexa Fluor® 750/IRDye® 800.

16. Substrat selon l'une quelconque des revendications 1 à 11,
**caractérisé par le fait que** le groupement I₁ est un groupement non fluorescent choisi parmi les composés vendus sous les dénominations commerciales DABCYL® et dérivés, Black Hole Quencher®, Nanogold Particules®, Eclipse Dark Quencher®, Elle Quencher®, Cy7Q, FluoQuench® et les colorants QSY®.

17. Substrat selon l'une quelconque des revendications précédentes;
**caractérisé par le fait qu'**il est choisi parmi les composés dans lesquels :
i) le squelette S est une galactosamine, F₁ et I₁, identiques et sont choisis parmi les groupements Cy5, Alexa Fluor® 750, Cy7 et IRDye® 800, ces groupements étant respectivement fixés en positions 1 et 2 de la galactosamine ;
ii) le squelette S est une galactosamine, F₁ est un groupement Cy5, Alexa Fluor® 750, Alexa Fluor ® 633 ou IRDye® 800 et I₁ est un groupement QSY ® 21, Cy7Q ou BHQ 3 ;
iii) le squelette S est une lactosamine, F₁ est un groupement Cy5, Cy7, Alexa Fluor® 750 ou IRDye® 800 et I₁ est un groupement QSY® 21, Cy7Q
ou BHQ 3 ;
iv) le squelette S est un trisaccharide comportant une glucosamine en position terminale et dont les deux autres unités saccharidiques, identiques ou différente, sont choisies parmi le galactose et le fucose, F₁ est un groupement Cy5, Cy7, Alexa Fluor® 750 ou IRDye® 800 et I₁ est un groupement QSY® 21, Cy7Q
ou BHQ 3.

18. Utilisation d'au moins un substrat enzymatique de structure (I) tel que défini à l'une quelconque des revendications 1 à 17, à titre de réactif fluorescent pour la détection d'une activité enzymatique *in vitro.*

19. Utilisation d'au moins un substrat enzymatique de structure (I) tel que défini à l'une quelconque des revendications 1 à 17 et dans lequel le groupement fluorophore F₁ est choisi parmi les groupements absorbant et émettant à une longueur d'onde comprise entre 640 et 900 nm, pour la préparation d'un réactif de diagnostic destiné à l'imagerie fonctionnelle *in vivo*.

20. Utilisation selon la revendication 19, **caractérisée par le fait que** ledit réactif est destiné à imager, par fluorescence, l'expression des gènes rapporteurs LacZ et gusA de *E. coli*.

21. Réactif de diagnostic, **caractérisé par le fait qu'**il comprend au moins une solution constituée d'eau ou d'un mélange d'eau et d'au moins un solvant organique, ladite solution renfermant au moins un substrat enzymatique de structure (I) tel que défini à l'une quelconque des revendications 1 à 17.

22. Réactif selon la revendication 21, **caractérisé par le fait qu'**il s'agit d'un réactif de diagnostic *in vivo* et que le substrat enzymatique de structure (I) comprend au moins un groupement fluorophore F₁ choisi parmi les groupements fluorophores absorbant et émettant dans le proche infrarouge.

23. Réactif selon la revendication 21 ou 22, **caractérisé par le fait que** la concentration du ou des substrats enzymatiques de structure (I) est comprise entre 1 µM et 1 mM.

## Claims

1. Fluorescent enzymatic substrate, **characterized in that** it has the following structure (I) 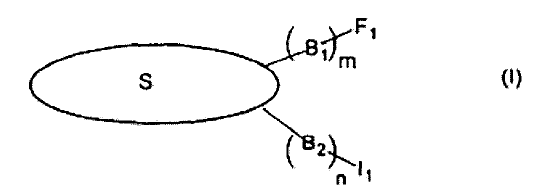 in which
- S is a skeleton of a saccharide nature made up of at least one saccharide unit chosen from monosaccharides, oligosaccharides having from 2 to 9 saccharide units and polysaccharides having at least 10 saccharide units;
- B₁ and B₂ are identical or different and represent a spacer arm made up of a linear or branched, saturated or unsaturated, optionally substituted hydrocarbon chain interrupted and/or terminated by one or more heteroatoms chosen from N, O or S, and/or by one or more groups chosen from C₁-C₄ alkyl, C₁-C₄ alkoxy or aryl radicals or by one or more functions chosen from the ether, ester, amide, carbonyl, carbamate, urea, thiourea and disulfide functions;
- m and n independently of one another are integers equal to 0 or 1;
- F₁ is a fluorophore group;
- I₁ is an inhibitor of the fluorescence of F₁;
i) F₁ and I₁, either directly or by the intermediary of the spacer arms B₁ and/or B₂ if m and/or n = 1, are both grafted onto the same saccharide unit of the skeleton S;
ii) one of the groups F₁ and I₁ is grafted in the anomeric position 1 of the said saccharide unit, the anomeric bond being in either position α or position β, the other group F₁ or I₁ occupying any free position of the same saccharide unit; and
iii) no phosphate chain, saccharide unit or nitrogen base may be inserted between the saccharide unit of the skeleton S carrying the groups F₁ and I₁ and the said groups F₁ and I₁.

2. Substrate according to claim 1, **characterized in that** the saccharide units of the skeleton S of the substrates of structure (I) are chosen from galactose, mannose, idose, talose, rhamnose, glucose, ribose, fucose and their amine or acid derivatives.

3. Substrate according to claim 2, **characterized in that** the amine and acid derivatives of the saccharide units are chosen from galactosamine, glucosamine, lactosamine, glucuronic acid, iduronic acid and sialic acid.

4. Substrate according to claim 2 or 3, **characterized in that** the saccharide units of the skeleton S of the substrates of structure (I) are chosen from glucosamine, galactose and glucuronic acid.

5. Substrate according to any one of the preceding claims, **characterized in that** the skeleton S is an oligosaccharide chosen from the oligosaccharides containing from 4 to 9 saccharide units.

6. Substrate according to any one of claims 1 to 5, **characterized in that** when the groups F₁ and/or I₁ are bonded directly to the terminal saccharide unit of the skeleton S, the group F₁ or I₁ in the anomeric position 1 of the saccharide unit is bonded to the latter by the intermediary of a covalent bond involving at least one atom X chosen from oxygen, carbon, sulfur and nitrogen atoms.

7. Substrate according to any one of the preceding claims, **characterized in that** the free positions of saccharide units of the skeleton S which contain neither the fluorophore group F₁ nor the inhibitor group I₁ and which are not involved in a glycosidic bond can be either unsubstituted (-H or -OH) or substituted by an amine function or by a group resulting from interaction of a hydroxyl function or an amine function with a protective group.

8. Substrate according to any one of the preceding claims, **characterized in that** the fluorophore group is chosen from fluorescein and its derivatives; fluorescent dyestuffs which absorb and emit at a wavelength of between 640 and 900 nm; Cy5 (n = 2) and Cy7 (n = 3); 7-hydroxy-9H-(1,3-dichloro-9,9-dimethylacridin-2-one), rhodamine and its derivatives; fluorescent dyestuffs with reactive amines; fluorescent dyestuffs marketed under the commercial names BODIPY®, IRDye® 800, Alexa Fluor® 750 and Alexa Fluor® 633; porphyrins; cyanines; oxazines and fluorescent nanoparticles.

9. Substrate according to claim 8, **characterized in that** the fluorophore group is chosen from the fluorophore groups which absorb and emit at a wavelength of between 640 and 900 nm.

10. Substrate according to claim 9, **characterized in that** the group F₁ is chosen from the fluorescent dyestuffs marketed under the names Fluorescent Red NIR 700 and Fluorescent Red NIR 730, Cy5 (n = 2), Cy7 (n = 3), 7-hydroxy-9*H*-(1,3-dichloro-9,9-dimethylacridin-2-one) (DDAO), IRDye® 800, Alexa Fluor® 750 and Alexa Fluor® 633.

11. Substrate according to any one of the preceding claims, **characterized in that** the fluorophore group F₁ is furthermore functionalized by one or more groups chosen from lipophilic chains, phospholipids and peptides.

12. Substrate according to any one of the preceding claims, **characterized in that** the group I₁ is a fluorescent group which inhibits or reduces or causes the complete disappearance of the fluorescence of the group F₁.

13. Substrate according to claim 12, **characterized in that** the group I₁ inhibits the fluorescence of F₁, is identical to the group F₁ and is chosen from cyanines and the fluorophore groups Cy5, Cy7, IRDye® 800, Alexa Fluor® 750 and Alexa Fluor® 633.

14. Fluorescent substrate according to claim 12,
**characterized in that** the group I₁ is a fluorescent group different from the group F₁ and which absorbs at the wavelength at which the group F₁ emits a fluorescence by fluorescence resonance energy transfer.

15. Fluorescent substrate according to claim 14,
**characterized in that** the F₁/I₁ pairs are chosen from the pairs Cy5/Cy7; Cy5/Alexa Fluor® 750; Alexa Fluor® 633/Cy7; Alexa Fluor® 633/Alexa Fluor® 750; Cy7/IRDye® 800 and Alexa Fluor® 750/IRDye® 800.

16. Substrate according to any one of claims 1 to 11, **characterized in that** the group I₁ is a non-fluorescent group chosen from the compounds marketed under the commercial names DABCYL® and derivatives, Block Hole Quencher®, Nanogold Particules®, Eclipse Dark Quencher®, Elle Quencher®, Cy7Q, FluoQuench® and QSY® dyestuffs.

17. Substrate according to any one of the preceding claims, **characterized in that** it is chosen from the compounds in which:
i) the skeleton S is a galactosamine, F₁ and I₁ are identical and are chosen from the groups Cy5, Alexa Fluor® 750, Cy7 and IRDye® 800, these groups being attached in positions 1 and 2 respectively of the galactosamine;
ii) the skeleton S is a galactosamine, F₁ is a Cy5, Alexa Fluor® 750, Alexa Fluor® 633 or IRDye® 800 group and I₁ is a QSY® 21, Cy7Q or BHQ 3 group;
iii) the skeleton S is a lactosamine, F₁ is a Cy5, Cy7, Alexa Fluor® 750 or IRDye® 800 group and I₁ is a QSY® 21, Cy7Q or BHQ 3 group;
iv) the skeleton S is a trisaccharide containing a glucosamine at the terminal position and of which the other two saccharide units are identical or different and are chosen from galactose and fucose, F₁ is a Cy5, Cy7, Alexa Fluor ® 750 or IRDye® 800 group and I₁ is a QSY® 21, Cy7Q or BHQ 3 group.

18. Use of at least one enzymatic substrate of structure (I) as defined in any one of claims 1 to 17 as a fluorescent reagent for detection of an enzymatic activity *in vitro*.

19. Use of at least one enzymatic substrate of structure (I) as defined in any one of claims 1 to 17, in which the fluorophore group F₁ is chosen from the groups which absorb and emit at a wavelength of between 640 and 900 nm, for the preparation of a diagnostic reagent for functional imaging *in vivo.*

20. Use according to claim 19, **characterized in that** the said reagent is for fluorescence imaging of the expression of the reporter genes LacZ and gusA of *E*. *coli.*

21. Diagnostic reagent, **characterized in that** it contains at least one solution made up of water or a mixture of water and at least one organic solvent, the said solutions containing at least one enzymatic substrate of structure (I) as defined in any one of claims 1 to 17.

22. Reagent according to claim 21, **characterized in that** it is an *in vivo* diagnostic reagent and **in that** the enzymatic substrate of structure (I) contains at least one fluorophore group F₁ chosen from the fluorophore groups which absorb and emit in the near infrared.

23. Reagent according to claim 21 or 22, **characterized in that** the concentration of the enzymatic substrate or substrates of structure (I) is between 1 µM and 1 mM.

## Patentansprüche

1. Fluoreszierendes Enzymsubstrat, **dadurch gekennzeichnet, dass** es der folgenden Struktur (I) entspricht: 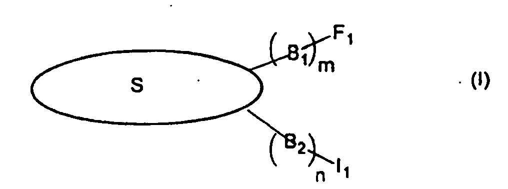 wobei:
- S ein Saccharidgerüst ist, das wenigstens eine Saccharideinheit umfasst und ausgewählt ist aus Monosacchariden, Oligosacchariden mit 2 bis 9 Saccharideinheiten und Polysacchariden mit wenigstens 10 Saccharideinheiten;
- B₁ und B₂, die identisch oder verschieden sein können, einen Spacerarm darstellen, der eine Kohlenwasserstoffkette umfasst, die linear oder verzweigt, gesättigt oder ungesättigt, und gegebenenfalls durch ein oder mehrere Heteroatome, ausgewählt aus N, O oder S, und/oder ein oder mehrere Gruppen, ausgewählt aus C₁-C₄-Alkylresten, C₁-C₄-Alkoxyresten und Arylresten, oder ein oder mehrere funktionellen Gruppen, ausgewählt aus Ether-, Ester-, Amid-, Carboxyl-, Carbamat-, Harnstoff-, Thioharnstoff- und Disulfidfunktionen substituiert, unterbrochen und/oder terminiert sind;
- m und n unabhängig voneinander ganze Zahlen sind, die gleich 0 oder 1 sind;
- F₁ eine Fluorophorgruppe ist;
- I₁ ein Inhibitor der Fluoreszenz von F₁ ist;
wobei:
i) F₁ und I₁, entweder direkt oder über die Spacerarme B₁ und/oder B₂, wenn m und/oder n = 1, beide auf dieselbe Saccharideinheit des Gerüsts S gepfropft sind;
ii) eine der Gruppen F₁ und I₁ in der anomeren Position 1 der Saccharideinheit aufgepfropft ist, wobei die anomere Bindung sich in der α- oder β-Position nicht unterscheidet, und die andere Gruppe F₁ oder I₁ eine beliebige freie Position derselben Saccharideinheit besetzt; und
iii) keine Phosphatkette, Saccharideinheit oder Stickstoffbase zwischen der Saccharideinheit des Gerüsts S, das die Gruppen F₁ und I₁ trägt, und die Gruppen F₁ und I₁ interkaliert.

2. Substrat nach Anspruch 1, **dadurch gekennzeichnet, dass** die Saccharideinheiten des Gerüsts S der Substrate der Struktur (I) ausgewählt sind aus Galactose, Mannose, Idose, Talose, Rhamnose, Glucose, Ribose, Fucose und deren Amino- oder Säurederivaten.

3. Substrat nach Anspruch 2, **dadurch gekennzeichnet, dass** die Amino- und Säurederivate der Saccharideinheiten ausgewählt sind aus Galactosamin, Glucosamin, Lactosamin, Glucuronsäure, Iduronsäure und Sialinsäure.

4. Substrat nach Anspruch 2 oder 3, **dadurch gekennzeichnet, dass** die Saccharideinheiten des Gerüsts S der Substrate der Struktur (I) ausgewählt sind aus Glucosamin, Galactose und Glucuronsäure.

5. Substrat nach einem der vorhergehenden Ansprüche, **dadurch gekennzeichnet, dass** das Gerüst S ein Oligosaccharid ist, ausgewählt aus Oligosacchariden, die 4 bis 9 Saccharideinheiten umfassen.

6. Substrat nach einem der Ansprüche 1 bis 5, **dadurch gekennzeichnet, dass** wenn die Gruppen F₁ und/oder I₁ direkt an die endständige Saccharideinheit des Gerüsts S gebunden sind, die Gruppe F₁ oder I₁, die sich in der anomeren Position 1 der Saccharideinheit befindet, an diese letztere über eine kovalente Bindung gebunden ist, die wenigstens ein Atom X umfasst, ausgewählt aus Sauerstoff-, Kohlenstoff-, Schwefel- oder Stickstoffatomen.

7. Substrat nach einem der vorhergehenden Ansprüche, **dadurch gekennzeichnet, dass** die freien Positionen der Saccharideinheiten des Gerüsts S, die weder die Fluorophorgruppe F₁ noch die Inhibitorgruppe I₁ umfassen, und die nicht an einer glycosidischen Bindung beteiligt sind, unabhängig voneinander unsubstituiert (-H oder -OH) sein können oder mit einer Aminofunktion oder mit einer Gruppe, die aus der Wechselwirkung mit einer Hydroxylfunktion oder einer Aminofunktion mit einer Schutzgruppe resultiert, substituiert sein können.

8. Substrat nach einem der vorhergehenden Ansprüche, **dadurch gekennzeichnet, dass** die Fluorophorgruppe ausgewählt ist aus Fluorescein und seinen Derivaten; fluoreszierenden Farbstoffen, die bei einer Wellenlänge zwischen 640 und 900 nm absorbieren und emittieren; Cy5 (n = 2) und Cy7 (n = 3); 7-Hydroxy-9H-(1,3-dichlor-9,9-dimethylacridin-2-on), Rhodamin und seinen Derivaten; fluoreszierenden Farbstoffen, die reaktive Amine umfassen; fluoreszierenden Farbstoffen, die unter den Handelsnamen BODIPY®, IRDye® 800, Alexa Fluor® 750 und Alexa Fluor® 633 verkauft werden; Porphyrinen, Cyaninen, Oxazinen und fluoreszierenden Nanopartikeln.

9. Substrat nach Anspruch 8, **dadurch gekennzeichnet, dass** die Fluorophorgruppe ausgewählt ist aus Fluorophorgruppen, die bei einer Wellenlänge zwischen 640 und 900 nm absorbieren und emittieren.

10. Substrat nach Anspruch 9, **dadurch gekennzeichnet, dass** die Gruppe F₁ ausgewählt ist aus fluoreszierenden Farbstoffen, die unter den Bezeichnungen Fluorescent Red NIR 700 und Fluorescent Red NIR 730, Cy5 (n = 2), Cy7 (n = 3), 7-Hydroxy-9H-(1,3-dichlor-9,9-dimethylacridin-2-on) (DDAO), IRDye® 800, Alexa Fluor® 750 und Alexa Fluor® 633 verkauft werden.

11. Substrat nach einem der vorhergehenden Ansprüche, **dadurch gekennzeichnet, dass** die Fluorophorgruppe F₁ weiterhin mit einer oder mehreren Gruppen ausgewählt aus lipophilen Ketten, Phospholipiden und Peptiden funktionalisiert ist.

12. Substrat nach einem der vorhergehenden Ansprüche, **dadurch gekennzeichnet, dass** die Gruppe I₁ eine fluoreszierende Gruppe ist, die die Fluoreszenz der Gruppe F₁ inhibiert, verringert oder deren vollständiges Verschwinden verursacht.

13. Substrat nach Anspruch 12, **dadurch gekennzeichnet, dass** die Gruppe I₁, die die Fluoreszenz von F₁ inhibiert, identisch ist mit der Gruppe F₁ und ausgewählt ist aus Cyaninen, den Fluorophorgruppen Cy5, Cy7, IRDye ® 800, Alexa Fluor® 750 und Alexa Fluor ® 633.

14. Fluoreszierendes Substrat nach Anspruch 12, **dadurch gekennzeichnet, dass** die Gruppe I₁ eine fluoreszierende Gruppe ist, die von der Gruppe F₁ verschieden ist und durch Fluoreszenzresonanz-Energietransfer bei einer Wellenlänge absorbiert, bei der die Gruppe F₁ Fluoreszenz emittiert.

15. Fluoreszierendes Substrat nach Anspruch 14, **dadurch gekennzeichnet, dass** die Paare F₁/I₁ ausgewählt sind aus den Paaren Cy5/Cy7; Cy5/Alexa Fluor ® 750; Alexa Fluor ® 633/Cy7; Alexa Fluor ® 633/Alexa Fluor ® 750; Cy7//RDye ® 800 und Alexa Fluor ® 750/IRDye ® 800.

16. Substrat nach einem der Ansprüche 1 bis 11, **dadurch gekennzeichnet dass** die Gruppe I₁ eine nichtfluoreszierende Gruppe ist, ausgewählt aus den Zusammensetzungen, die unter den Handelsnamen DABCYL® und dessen Derivate, Black Hole Quencher ®, Nanogold Partikel ®, Eclipse Dark Quencher ®, Elle Quencher ®, Cy7Q, FluoQuench ® und die Farbstoffe QSY ® verkauft werden.

17. Substrat nach einem der vorhergehenden Ansprüche, **dadurch gekennzeichnet, dass** es ausgewählt ist aus Verbindungen, bei denen:
i) Gerüst S ein Galactosamin ist, F₁ und I₁ identisch sind und ausgewählt sind aus den Gruppen Cy5, Alexa Fluor ® 750, Cy7 und IRDye ® 800, wobei diese Gruppen jeweils an den Positionen 1 und 2 des Galactosamins fixiert sind;
ii) Gerüst S ein Galactosamin ist, F₁ die Gruppe Cy5, Alexa Fluor ® 750, Alexa Fluor ® 633 oder IRDye ® 800 ist und I₁ die Gruppe QSY ® 21, Cy7Q oder BHQ 3 ist;
iii) Gerüst S ein Lactosamin ist, F₁ die Gruppe Cy5, Cy7, Alexa Fluor ® 750 oder IRDye ® 800 ist und I₁ die Gruppe QSY ® 21, Cy7Q oder BHQ 3 ist;
iv) Gerüst S ein Trisaccharid ist, das in endständiger Position ein Glucosamin umfasst und deren beiden anderen Saccharidenheiten, die identisch oder verschieden sein können, ausgewählt sind aus Galactose und Fucose, F₁ die Gruppe Cy5, Cy7, Alexa Fluor ® 750 oder IRDye ® 800 ist und I₁ die Gruppe QSY ® 21, Cy7Q oder BHQ 3 ist.

18. Verwendung wenigstens eines Enzymsubstrats der Struktur (I) nach einem der Ansprüche 1 bis 17 als fluoreszierendes Reagenz zum Nachweis einer Enzymaktivität *in vitro.*

19. Verwendung wenigstens eines Enzymsubstrats der Struktur (I) nach einem der Ansprüche 1 bis 17, deren Fluorophorgruppe F₁ ausgewählt ist aus den Gruppen, die bei einer Wellenlänge absorbieren oder emittieren, die zwischen 640 und 900 nm liegt, zur Herstellung eines Diagnosereagenzes zur funktionellen Bildgebung in *vivo.*

20. Verwendung nach Anspruch 19, **dadurch gekennzeichnet, dass** das Reagenz zur Abbildung der Expression der *E*.*coli*-LacZ- und gusA-Reportergene bestimmt ist.

21. Diagnosereagenz, **dadurch gekennzeichnet, dass** es wenigstens eine Lösung umfasst, die Wasser oder eine Mischung aus Wasser und wenigstens einem organischen Lösungsmittel umfasst, wobei die Lösung wenigstens ein Enzymsubstrat der Struktur (I) nach einem der Ansprüche 1 bis 17 umfasst.

22. Reagenz nach Anspruch 21, **dadurch gekennzeichnet, dass** es ein *in vivo* Diagnosereagenz ist und dass das Enzymsubstrat der Struktur (I) wenigstens eine Fluorophorgruppe F₁ umfasst, ausgewählt aus den Fluorophorgruppen, die im nahen Infrarot absorbieren und emittieren.

23. Reagenz nach Anspruch 21 oder 22, **dadurch gekennzeichnet, dass** die Konzentration des oder der Enzymsubstrate der Struktur (I) zwischen 1 µM und 1 mM ist.
